# EUROPÄISCHE PATENTSCHRIFT

(11) **EP 3 858 230 B1**
(45) Veröffentlichungstag und Bekanntmachung des Hinweises auf die Patenterteilung: **04.11.2026**
(21) Anmeldenummer: 21154615.5
(22) Anmeldetag: 01.02.2021
(51) Int. Cl.: A61B 5/024, A61B 5/0537, A61B 5/107, A61B 5/1455, A61B 5/26, A61B 5/282, G01G 19/50

(54) **VORRICHTUNG ZUR BESTIMMUNG WENIGSTENS EINES GESUNDHEITSPARAMETERS EINER PERSON**
DEVICE FOR DETERMINING AT LEAST ONE HEALTH PARAMETER OF A PERSON
DISPOSITIF DE DÉTERMINATION D'AU MOINS UN PARAMÈTRE DE SANTÉ D'UNE PERSONNE

(30) Priorität: 03.02.2020 DE 102020102663
(43) Veröffentlichungstag der Anmeldung: 04.08.2021
(73) Patentinhaber: Cardioscan GmbH, 22761 Hamburg (DE)
(72) Erfinder: RENNIES, Sven, 20144 Hamburg (DE); SEEBECK, Johann, 22391 Hamburg (DE)
(74) Vertreter: Holz, Christian

(56) Entgegenhaltungen:
- EP-A1- 0 998 875
- CN-A- 108 992 070
- JP-A- 2003 265 429
- KR-A- 20010 019 513
- KR-A- 20010 106 961
- US-A1- 2016 015 276

## Beschreibung

Vorrichtung zur Bestimmung wenigstens eines Gesundheitsparameters einer Person

Die vorliegende Erfindung betrifft eine Vorrichtung zur Bestimmung wenigstens eines Gesundheitsparameters einer Person.

Es ist bekannt, Menschen hinsichtlich ihrer Gesundheit medizinisch zu untersuchen sowie zu überwachen. Neben der medizinischen Behandlung kann dies auch der Vorbeugung von Krankheiten und anderen gesundheitlichen Einschränkungen dienen. Auch können mittels derartiger medizinischer Untersuchungen Informationen über den Gesundheitszustand einer Person gewonnen werden, um ihre sportliche Entwicklung durch Training, Ernährung und Regeneration unterstützend begleiten zu können. Ferner können derartige Informationen zur Unterstützung einer gesunden bzw. einer gesundheitsfördernden Ernährung und Regeneration der Person verwendet werden. In jedem Fall erfolgt eine Aussage zum aktuellen Gesundheitszustand der Person basierend auf einem gesundheitsrelevantem Parameter oder basierend auf mehreren gesundheitsrelevanten Parametern, welcher bzw. welche messtechnisch erfasst und einzeln und oder in Kombination ausgewertet wird bzw. werden.

Ein vergleichsweise einfach zu bestimmender gesundheitsrelevanter Parameter einer Person stellt das Gewicht der Person dar, welches beispielsweise mit einer Waage messtechnisch bestimmt werden kann, indem die Person eine Plattform der Körperwaage betritt. Das Gewicht der Person wird dabei üblicherweise von der Plattform auf eine Federwage mit Dehnungsmesstreifen übertragen, und mittels geeigneter Messelektronik und Wandler erfasst sowie ausgewertet.

Ein weiterer vergleichsweise einfach erfassbarer Parameter einer Person, welcher im Zusammenhang mit gesundheitsrelevanten Paramatern ermittelt werden kann, ist die Größe der Person, welche beispielsweise mittels eines Maßbandes oder eines an einer Wand montierten und in der vertikalen Richtung ausgerichteten Höhenmessgerätes erfolgen kann.

Darüber hinaus ist auch die Ermittlung der Körperzusammensetzung einer Person durch die bioelektrische Impedanzanalyse (BIA) bekannt, bei welcher mit einem konstanten Signal eines Wechselstroms in Höhe von ca. 0,8 mA bei einer Frequenz von ca. 50 kHz der ohmsche Wechselstromwiderstand, auch ohmsche Impedanz genannt, des Körpers der Person sensorisch erfasst. Beispielsweise bei einer tetrapolaren bioelektrischen Impedanzanalyse wird über zwei äußere Elektroden ein elektromagnetisches Feld im Körper der Person aufgebaut und gleichzeitig über zwei weitere Elektroden im Inneren dieses Feldes der Spannungsabfall und die Phasenverschiebung der Signalspannung als Vierleitermessung gemessen. Aus den erfassten Werten des Spannungsabfalls und der Phasenverschiebung können als Parameter der Körperzusammensetzung der Person zum Beispiel dessen Fettmasse (Fat mass - FM), dessen Körperwasser (Total body water - TBW), dessen Muskelmasse (MM - Muscle Mass), dessen fettfreie Masse (Fat free mass - FFM), dessen Körperzellmasse (Body cell mass - BCM), dessen extrazelluläre Masse (Extracellular mass - ECM) sowie dessen Magermasse (Lean body mass - LBM) bestimmt werden.

Nachteilig bei der bioelektrischen Impedanzanalyse ist, dass für eine gültige und wiederholbare BIA-Messung die Positionierung der Elektroden an dieser "inneren Messstrecke" genau zu beachten ist. Andernfalls kann es bereits bei geringen Abweichungen der Positionierung der Elektroden zu fehlerhaften und bzw. oder nicht reproduzierbaren Messungen des Spannungsabfalls und der Phasenverschiebung kommen, aus denen ungültige bzw. unbrauchbare Parameter resultieren können. Dies gilt ebenso für bereits geringe Abweichungen der Körperposition zwischen den sich wiederholenden Messungen und insbesondere bei einem abweichenden Winkel zwischen Arm und Torso, da dies zu einer Änderung der "inneren Messstrecke" aufgrund einer Verkürzung des Strompfades führt.

Daher setzen international vereinbarte, wissenschaftliche Standardisierungen für die bioelektrische Impedanzanalyse eine definierte Messpositionierung voraus. Die zu vermessende Person liegt rücklings entspannt in der Waagerechten und die Gliedmaßen sind vom Rumpf leicht abgewinkelt. Auch sind Vorrichtungen bekannt, bei denen definierte Messpositionen von einer aufrechtstehenden Person eingenommen werden können. In jedem Fall ist die Platzierung der Elektroden genauestens vorgegeben und zu beachten, weshalb die Durchführung einer bioelektrischen Impedanzanalyse in jedem Fall lediglich durch eingewiesenes und geschultes Messpersonal erfolgen sollte. Dies führt jedoch zu einem erhöhten Aufwand und zu erhöhten Kosten der bioelektrischen Impedanzanalyse, welche in dieser Art lediglich durch medizinische Einrichtung durchgeführt werden kann. Auch schränkt dies die Verfügbarkeit der BIA-Messung für die breite Öffentlichkeit ein. Wird auf die Unterstützung von geschultem Personal verzichtet, so kann dies die Zuverlässigkeit der Messergebnisse reduzieren.

Ebenfalls zur Ermittlung von Gesundheitsparametern ist die Erstellung von Elektrokardiogrammen (EKG) bekannt. Hierunter wird die Aufzeichnung der Summe der elektrischen Aktivitäten aller Herzmuskelfasern mittels eines Elektrokardiografen, kurz EKG-Gerät, verstanden. Die Durchführung dieser Aufzeichnung kann auch als Elektrokardiographie bezeichnet werden. Es wird sich hierbei zu Nutze gemacht, dass jeder Kontraktion des Herzmuskels eine elektrische Erregung vorausgeht. Diese elektrischen Spannungsänderungen am Herzen können an der Körperoberfläche sensorisch erfasst und über der Zeit dargestellt bzw. aufgezeichnet werden. Dieser zeitliche Verlauf stellt ein immer wiederkehrendes Bild der elektrischen Herzaktion der Person dar, aus welcher sich vielfältige Aussagen zu den Eigenschaften und zu der Gesundheit des Herzens der Person treffen lassen.

Zur sensorischen Erfassung der elektrischen Spannungsänderungen bei einer Elektrokardiographie können mehrere Elektroden an verschiedenen definierten, d.h. vorgegebenen, Positionen der Körperoberfläche der Person angebracht werden. Dies können zum Beispiel die Kombination aus rechtem und linkem Arm, aus rechtem Arm und linkem Bein oder aus linken Arm und linkem Bein aber auch Kombinationen von verschiedenen Punkten zum Beispiel im Bereich Brustbein, Schlüsselbein und Rippen sein.

Nachteilig ist somit auch bei der Elektrokardiographie, dass für gültige und wiederholbare Messungen die Elektroden stets an definierten Positionen der Körperoberfläche angeordnet werden müssen. Auch hier können Abweichungen der Positionierung der Elektroden zu fehlerhaften und bzw. oder zu nicht reproduzierbaren Messungen der elektrischen Spannungsänderungen am Herzen und damit zu ungültigen bzw. unbrauchbaren zeitlichen Verläufen führen.

Ferner ist zur Ermittlung von Gesundheitsparametern die Photoplethysmographie (PPG) und die Pulsoxymetrie bekannt. Im am weitesten verbreiteten Verfahren basieren diese Methoden auf der Erkenntnis, dass mit O2 beladenes Hämoglobin, d.h. oxygeniertes Hämoglobin (HbO2), bei bestimmten optischen Wellenlängen einen deutlich anderen Lichtabsorptionsverlauf als desoxygeniertes Hämoglobin (Hb) aufweist. Bei der Photopletysmographie steht die Erfassung der Form und zeitlichen Verläufe der Pulswellen im Vordergrund, welche primär die Kontraktion des Herzens, die Elastizität der Aorta, sowie der Struktur des gesamten Gefäßsystems abbilden. Die Pulsoxymetrie stellt hingegen die Auswertung der Sauerstoffsättigung des Blutes in den Vordergrund. Technisch wird bei beiden Verfahren die Haut mittels monochromatischer Leuchtdioden als Signallichtquelle durchleuchtet und die wellenlängenabhängige Absorption mittels entsprechender Fotodioden oder Laserdioden als Signallichtsensor sensorisch erfasst und anschließend ausgewertet. Zur Abschirmung von Fremdlicht wird die optische Einheit mit Signallichtquelle und Signallichtsensor üblicherweise innerhalb eines Gehäuses in Form eines Clips angeordnet, welcher Fremdlicht abschirmen kann. Der Clip wird üblicherweise an einem leicht zugänglichen Körperteil wie zum Beispiel an einem Finger bzw. an einer Fingerkuppe, an einem Zeh oder an einem Ohrläppchen angeordnet, um die Messung durchzuführen. Aus der Auswertung der erfassten Messwerte, welche im verbreitetsten Verfahren der Lichtabsorption des oxygenierten und desoxygenierten Hämoglobin entsprechen, können die arterielle Sauerstoffsättigung, der Puls sowie die Variabilität des Pulses der Person bestimmt werden.

Nachteilig ist sowohl bei der Photoplethysmographie als auch bei der Pulsoxymetrie, dass zur Durchführung der Messungen lediglich bestimmte Körperteile bzw. bestimmte Positionen an der Körperoberfläche in Frage kommen.

Nachteilig bei der bioelektrischen Impedanzanalyse (BIA), bei der Erstellung von Elektrokardiogrammen (EKG) sowie bei der Photoplethysmographie (PPG) ist, dass diese jeweils zur Ermittlung von einzelnen oder ggfs. mehreren Gesundheitsparametern verwendet werden können, jedoch leidglich diese Gesundheitsparameter ermittelt werden können. Sollen auch andere bzw. die übrigen zuvor beschriebenen Gesundheitsparameter sensorisch erfasst und bestimmt werden, so sind bisher die o.g. Verfahren mit unterschiedlichen Geräten nacheinander durchzuführen. Dies erhöht den Aufwand an Geräten sowie ggfs. an geschultem Personal ebenso wie die erforderliche Zeit, was für die zu vermessende Person störend sein und die Person ggfs. von der Durchführung der o.g. Verfahren abhalten kann. Dann stehen diese Gesundheitsparameter jedoch nicht zur Verfügung, um insbesondere einen Gesundheitszustand, einen Trainingszustand, einen Ernährungszustand, einen Erholungszustand und bzw. oder einen Regenerationszustand der Person mittels der zuvor beschriebenen Verfahren beurteilen zu können. Entsprechend weitreichende, ganzheitliche Rückschlüsse zur Gesundheit der vermessenden Person werden hierdurch verhindert bzw. erschwert.

Die DE 10 2011 118 810 A1 beschreibt eine Vorrichtung zur Messung der Körperlänge einer Person. Im Bereich einer Innenbegrenzung eines Gebäuderaumes sind mindestens ein Emitter zur Abgabe eines Primärsignals und mindestens ein Sensor zur Erfassung eines Messsignals angeordnet. Sowohl der Emitter als auch der Sensor sind mit einer Steuereinrichtung gekoppelt. Die Steuereinrichtung ist mit einer Anzeigeeinrichtung gekoppelt und zur Auswertung einer Referenzmessung ausgebildet, die den Abstand des Sensors zu einer Standfläche der zu vermessenden Person berücksichtigt. Die Vorrichtung weist zusätzlich eine Waage zur Erfassung eines Gewichtes der zu vermessenden Person auf.

Die WO 2019 211 335 A1 beschreibt eine Vorrichtung zur Bestimmung eines Stress- und bzw. oder Schmerzpegels eines Subjekts und umfasst einen Prozessor. Der Prozessor ist konfiguriert, um von einem Photoplethysmographiesensor ein Photoplethysmographiesignal zu erfassen, das von einem zentralen Teil des Körpers des Subjekts erhalten wird. Der Prozessor ist außerdem so konfiguriert, dass er eine Charakteristik des erfassten Photoplethysmographiesignals identifiziert. Die Charakteristik ist auf den Blutdruck normiert. Der Prozessor ist ferner so konfiguriert, dass er aus dem erfassten Photoplethysmographiesignal den Stress- und bzw. oder Schmerzpegel des Subjekts auf der Grundlage der normierten Charakteristik bestimmt.

Die DE 10 2010 023 122 A1 beschreibt eine Vorrichtung zur Messung von Bioimpedanzen, die jeweils mindestens eine Messelektrode für jeden Fuß und jede Hand einer zu vermessenden Person aufweist und bei der die Elektroden an eine Auswertungseinrichtung angeschlossen sind, sowie die mit mindestens einer Waage zur Bestimmung eines Körpergewichtes der zu vermessenden Person versehen ist.

Um hierbei eine korrekte Positionierung der Elektroden relativ zur zu vermessenden Person zu unterstützen, sind die für einen Kontakt mit den Händen der zu vermessenden Person vorgesehenen Elektroden im Bereich mindestens eines positionierbaren Tragelementes angeordnet. Hierdurch kann die Positionierung der Elektroden an die Körpergröße, an die konkrete Anatomie des Benutzers sowie an die Körperhaltung des Benutzers angepasst werden. Eine Anpassung an eine Körpergröße des Benutzers kann dadurch erfolgen, dass das Tragelement höhenpositionierbar angeordnet ist. Eine gespreizte Armhaltung wird dadurch unterstützt, dass das Tragelement mit einer horizontalen Richtungskomponente positionierbar ist. Eine angenehme Griffposition und bzw. oder eine Verstellung wird dadurch unterstützt, dass das Tragelement verdrehbar angeordnet ist. Vorgegebene Positionierbahnen können dadurch definiert werden, dass das Tragelement verschieblich entlang eines Führungselementes angeordnet ist.

Nachteilig bleibt bei den zuvor beschriebenen Vorrichtungen, dass falls diese Funktionen zur Bestimmung mehrerer zuvor beschriebener Gesundheitsparameter einer zu vermessenden Person aufweisen, dennoch mehrere der zuvor beschriebenen Gesundheitsparameter weiterhin nicht sensorisch erfasst und ausgewertet werden können.

Nachteilig ist bei der Vorrichtung der DE 10 2010 023 122 A1, dass zwar der zu vermessenden Person als Benutzer die Möglichkeit gegeben werden kann, die für den Kontakt mit den Händen der zu vermessenden Person vorgesehenen Elektroden korrekt, d.h. gemäß einer vorgegebenen Position sowie reproduzierbar, zu positionieren, dies jedoch von der zu vermessenden Person alleine durchgeführt werden muss. Dabei können die verschiedenen Möglichkeiten zur Veränderung der für einen Kontakt mit den Händen der zu vermessenden Person vorgesehenen Elektroden eher zur Verwirrung der zu vermessenden Person führen, da keine eindeutige und intuitive Position vermittelt wird. Insbesondere stehen derart umfangreiche Möglichkeiten zur Verfügung, die für den Kontakt mit den Händen der zu vermessenden Person vorgesehenen Elektroden relativ zur Person zu positionieren, dass die Einnahme einer korrekten und definierten Position und inbesondere einer reproduzierbaren Position für die Person durch die Vielzahl der Verstellmöglichkeiten sogar erschwert wird.

Zu beachten ist hierbei, dass es, wie zuvor bereits erwähnt, für die Ermittlung der Körperzusammensetzung durch die Bioelektrische Impedanzanalyse notwendig ist, dass die sich vermessende Person eine definierte und reproduzierbare Körperhaltung einnimmt. Hintergrund ist die sich verändere Impedanz des Körpers der Person bei verschiedenen Armhaltungen, welche zu verschiedenen Winkeln zwischen Arm und Torso führen, was keiner korrekten, definierten und reproduzierbaren Körperhaltung der Person entspricht.

Die verschiedenen Verstellmöglichkeiten der Vorrichtung der DE 10 2010 023 122 A1 stellen jedoch keinesfalls sicher, dass die vorgegebene Position dieser Elektroden von der Person richtig eingenommen wird, auch wenn die Verstellmöglichkeiten der Vorrichtung der DE 10 2010 023 122 A1 dies grundsätzlich ermöglichen können. Dies führt weiterhin zu den zuvor beschrieben Unsicherheiten bei der Nutzung der Vorrichtung der DE 10 2010 023 122 A1 und überwindet daher die eingangs beschriebenen Nachteile der bioelektrischen Impedanzanalyse nicht.

Selbst falls, trotz des damit verbundenen zusätzlichen Aufwands, geschultes Personal zur Nutzung der Vorrichtung der DE 10 2010 023 122 A1 eingesetzt wird, um aus den verschiedenen Verstellmöglichkeiten zur Veränderung der für einen Kontakt mit den Händen der zu vermessenden Person vorgesehenen Elektroden diejenige auszuwählen und einzustellen, welche für die jeweilige Person passend sein und einer vorangehenden Position, d.h. einer Position der zuletzt vorgenommenen Untersuchung, entsprechen kann, kann hierdurch dennoch nicht sichergestellt werden, dass die korrekte und definierte Position von der zu vermessenden Person eingenommen wird. Somit ist auch bei der Bedienung der Vorrichtung der DE 10 2010 023 122 A1 durch geschultes Personal eine Reproduzierbarkeit der Position der zu vermessenden Person, insbesondere bei wechselndem geschulten Personal und bzw. oder bei längeren Zeiträumen zwischen der aktuellen und der zuletzt vorgenommenen Untersuchung der zu vermessenden Person, nicht sichergestellt.

Die KR 2001 0106961 A beschreibt einen Körperzusammensetzungsanalysator, umfassend: einen Oberkörperteil mit einem Handelektrodenabschnitt, der Elektroden aufweist, die so angeordnet sind, dass sie beide Hände einer Person berühren, einen Unterkörperteil mit einem Generatorelektrodenabschnitt, der Elektroden aufweist, die so angeordnet sind, dass sie beide Füße berühren, eine Tastatur zur Eingabe von persönlichen Daten einschließlich der Körpergröße der Person, einen Impedanzmessabschnitt zur Messung eines Impedanzwertes des menschlichen Körpers unter Verwendung der Elektroden, einen Mikrocomputer zur Berechnung eines Körperzusammensetzungswertes der Person durch Vergleich des gemessenen Impedanzwertes mit den zuvor eingegebenen persönlichen Daten der Person, einen Bildschirm zur Anzeige der persönlichen Daten und des Körperzusammensetzungswertes, ein bewegliches Element, das den Oberkörperteil und den Unterkörperteil verbindet, eine in dem beweglichen Element vorgesehene motorische Höhenverstelleinrichtung zum Auf- und Abbewegen des beweglichen Elements, und eine Steuereinheit zur Steuerung der Höhenverstelleinrichtung gemäß der von der Person eingegebenen Körpergröße.

Die US 2016/015276 A1 beschreibt ein System zur Messung biometrischer Daten im Zusammenhang mit Herzinsuffizienz, wobei das System umfasst: eine Waage mit Fußelektroden und einer sich von der Waage erstreckenden Stütze, wobei die Stütze Haltegriffe mit Elektroden zur Messung biometrischer Daten aufweist, eine Benutzerschnittstelle zur Anzeige des Benutzerverlaufs, der Zielwerte für biometrische Daten und von Meldungen für einen Benutzer; und Mittel zum Starten der Messung einer Vielzahl von biometrischen Daten, zum Empfangen biometrischer Daten über einen vorbestimmten Zeitraum und zum Verarbeiten der biometrischen Daten. Die Haltegriffe sind gegenüber einem unteren Ende der Waage in der Höhe einstellbar.

Die CN 108 992 070 A beschreibt eine Mehrkamera-Vorrichtung zum dreidimensionalen Scannen des menschlichen Körpers. Die Vorrichtung umfasst eine feststehende Stützsäule, einen Drehtisch, eine Verbindungsplatte und Tiefenkameras. Die Mehrkamera-Vorrichtung umfasst ferner eine Zugstangen-Armlehne, die an einer Oberseite des Drehtisches angeordnet ist. Die Zugstangen-Armlehne umfasst einen höhenveränderlichen Griff und eine feststehende Sitzabdeckung, wobei eine Seitenwand der feststehenden Sitzabdeckung mit einer Öffnung versehen ist und die Seitenkante der Öffnung mit einer in vertikaler Richtung verlaufenden Verzahnung versehen ist. Die feststehende Sitzabdeckung ist mit einer Gleitschiene versehen, wobei ein Gleitabschnitt der Gleitschiene mit einem Push-Pull-Elektromagneten versehen ist und an einer Schubstange des Push-Pull-Elektromagneten ein Rastelement befestigt ist, das in den gezahnten Abschnitt eingreift. Der Push-Pull-Elektromagnet wird ein- und ausgeschaltet, um das Einrasten und Ausrasten des Rastelements und des gezahnten Abschnitts zu bewirken. Der Griff ist am oberen Ende des Gleitabschnitts befestigt.

Die EP 0 998 875 A1 beschreibt ein Gerät zur Messung des Körperfettanteils, das die In-vivo-Impedanz misst und den Körperfettanteil berechnet, mit einer Vorrichtung zur Messung der Körpergröße, die Folgendes umfasst: (1) eine Fußstütze, auf die sich eine zu untersuchende Person stellt, (2) eine Messklemme zum Kontaktieren der Kopfoberseite der zu untersuchenden Person und (3) einen Cursor, der in Aufwärts- und Abwärtsrichtung bewegbar ist und die Messklemme hält, sowie in den Cursor integrierte Elektroden zur Messung der In-vivo-Impedanz, wobei die In-vivo-Impedanz gemessen wird, wenn die zu untersuchende Person die Elektrode mit der Hand ergreift. Der Cursor ist entlang einer säulenförmigen Halterung in Aufwärts- und Abwärtsrichtung beweglich.

Die KR 2001 0019513 A beschreibt ein automatisches Analysegerät zur Bestimmung der Körperzusammensetzung umfassend: mehrere Elektroden, ein mit einem Griff verbundenes Höhenverstellelement, das den Griff nach oben und unten bewegt, einen linearen Sensor zur Messung der Körpergröße der zu untersuchenden Person durch elektronische Erfassung der Fallstrecke einer Messstange sowie einen Lastsensor zur Messung des Gewichts der Person. Das Analysegerät umfasst eine Impedanzbestimmungsschaltung, die Sinuswellen erzeugt und die Differenz zwischen den Elektroden misst, sowie einen elektronischen Schalter, der zwischen den Elektroden und der Impedanzschaltung angeordnet ist, um die Impedanzen der jeweiligen Körperregionen zu messen. Das Höhenverstellelement bewegt sich vertikal entlang einer linearen Führung hin- und her, so dass mittels des Höhenverstellelements eine Steuerung der Griffhöhe erfolgt.

Die JP 2003 265429 A beschreibt einen Körperzusammensetzungsanalysator, der mit einer Messbasis mit einem im Wesentlichen vertikalen Gehäuse versehen ist. An beiden Seiten des Gehäuses sind Griffe mit Handelektroden angebracht. Die Griffe sind über Universalgelenke mit dem Gehäuse verbunden, und die Griffe können in beliebigen Drehpositionen gehalten werden, sodass die Griffe frei sowohl in vertikaler Richtung als auch in horizontaler Richtung ge-dreht werden können.

Eine Aufgabe der vorliegenden Erfindung ist es, eine Vorrichtung zur Bestimmung wenigstens eines Gesundheitsparameters einer Person mittels einer bioelektrischen Impedanzanalyse der eingangs beschriebenen Art bereit zu stellen, so dass eine vorbestimmte Position bzw. eine vorbestimmte Körperhaltung der Person einfacher, verlässlicher und bzw. oder intuitiver als bisher bekannt eingenommen werden kann. Insbesondere soll dabei eine vorbestimmte Position bzw. eine vorbestimmte Körperhaltung der Hände und bzw. oder der Arme der Person einfacher, verlässlicher und bzw. oder intuitiver als bisher bekannt eingenommen werden können. Insbesondere sollen die bestimmbaren Gesundheitsparameter einer Person erweitert bzw. erhöht werden. In jedem Fall soll dies möglichst platzsparend, energiesparend, kostengünstig, zeitsparend, bedienerfreundlich und bzw. oder ohne zusätzliches Personal, insbesondere ohne geschultes Personal oder Fachperson, erfolgen können. Zumindest soll eine Alternative zu bekannten derartigen Vorrichtungen geschaffen werden.

### Zusammenfassung der Beschreibung

Die Aufgabe wird erfindungsgemäß durch eine Vorrichtung mit den Merkmalen des Patentanspruchs 1 gelöst. Vorteilhafte Weiterbildungen sind in den Unteransprüchen beschrieben.

### Zusammenfassung der Erfindung

Somit betrifft die vorliegende Offenbarung eine Vorrichtung zur Bestimmung wenigstens eines Gesundheitsparameters einer Person. Die Vorrichtung weist eine erste Fußelektrode und eine zweite Fußelektrode auf, welche ausgebildet sind, jeweils von einem Fuß der Person elektrisch kontaktiert zu werden. Die Vorrichtung weist eine erste Handelektrode und eine zweite Handelektrode auf, welche ausgebildet sind, jeweils von einer Hand der Person elektrisch kontaktiert zu werden. Die Handelektroden sind in der vertikalen Richtung höhenverstellbar ausgebildet. Eine Steuerungseinheit der Vorrichtung ist ausgebildet, mittels der Fußelektroden und mittels der Handelektroden Sensorwerte zur Durchführung einer bioelektrischen Impedanzanalyse zu erfassen.

Die Vorrichtung ist dadurch gekennzeichnet, dass die Handelektroden ferner ausgebildet sind, jeweils von einer Hand der Person gegriffen zu werden, wobei die Handelektroden ferner ausgebildet sind, in der vertikalen Richtung in einer ersten, unteren Position, die einen unteren Endanschlag der Handelektrode darstellt, aufgrund ihres Eigengewichts und der Schwerkraft von alleine einzunehmen und dort zu ruhen, so dass die Handelektroden von der Person in einer gebeugten Körperhaltung zu greifen sind, und von der Person in der vertikalen Richtung in eine zweite, obere Position bewegt zu werden, in welcher die Person in einer aufrechten Körperhaltung steht. Eine gebeugte Körperhaltung schließt auch eine Körperhaltung ein, in welcher die Person in die Knie geht.

Unter einem Gesundheitsparameter ist ein Parameter zu verstehen, welcher für sich alleine oder in Kombination mit weiteren Gesundheitsparametern betrachtet Aufschlüsse über die Gesundheit einer Person, d.h. über einen Menschen bzw. über einen menschlichen Körper, als biologisches Subjekt geben kann. Ein Gesundheitsparameter kann insbesondere das Gewicht, die Größe, ein Parameter der Körperzusammensetzung, ein EKG, die arterielle Sauerstoffsättigung, der Puls, die Variabilität des Pulses und dergleichen sein. Aus einem dieser Parameter und insbesondere aus der kombinierten Betrachtung bzw. Analyse mehrerer dieser Parameter können Rückschlüsse auf den Gesundheitszustand der Person gezogen werden. Hierzu kann es auch gehören, Rückschlüsse auf einen sportlichen Trainingszustand, auf einen einen Ernährungszustand und bzw. oder auf einen Regenerationszustand der Person ziehen zu können. Diese Informationen bzw. Erkenntnisse können dazu verwendet werden, Empfehlungen zur Verbesserung der Gesundheit, des Trainings, der Ernährung und bzw. oder der Regeneration der Person zu geben und entsprechende Verbesserungen zu erzielen.

Die Fußelektroden der Vorrichtung sind entsprechend so angeordnet, dass sich die zu vermessende Person mit beiden Füßen auf die beiden Fußelektroden in der vertikalen Richtung von oben draufstellen kann. Dies kann insbesondere in einer gebeugten oder aufrechtstehenden Körperhaltung der Person erfolgen. In jedem Fall kann die Person durch das Draufstellen mit dem einen Fuß auf die eine Fußelektrode der Vorrichtung und mit dem anderen Fuß auf die andere Fußelektrode der Vorrichtung jeweils die Fußelektroden kontaktieren und hierdurch jeweils eine elektrisch leitfähige Verbindung zwischen den Elektroden und der jeweiligen Fußsohle herstellen.

Vergleichbar sind die Handelektroden der Vorrichtung entsprechend so angeordnet, dass die zu vermessende Person mit den beiden Händen die beiden Handelektroden greifen und hierdurch jeweils ebenso eine elektrisch leitfähige Verbindung zwischen der jeweiligen Handelektrode und der jeweiligen Hand und insbesondere zwischen der Handinnenfläche bzw. zwischen der Innenseiten der Finger der jeweiligen Hand herstellen kann. Die beiden Handelektroden können jeweils derart von der Person gegriffen werden, dass die Handelektroden selbst oder ein entsprechendes Handelement, welches die Handelektroden aufweist, von der Person mit der Handfläche und mit den Fingern der jeweiligen Hand umgriffen werden können.

Unter Verwendung der beiden Fußelektroden und der beiden Handelektroden können nun seitens der Vorrichtung wenigstens Sensorwerte zur Durchführung einer bioelektrischen Impedanzanalyse erfasst werden, wie sie bekannt ist und eingangs beschrieben wurde. Diese Sensorwerte können außerhalb der Vorrichtung, zum Beispiel auf einem separaten Rechner, in einer Cloud und bzw. oder auf einem mobilen Endgerät wie zum Beispiel in einem Tablet oder in einem Smartphone verarbeitet und hierdurch eine bioelektrischen Impedanzanalyse durchgeführt werden, dessen Ergebnisse, d.h. die mittels der bioelektrischen Impedanzanalyse ermittelten Gesundheitsparameter und ggfs. hieraus resultierende bzw. hierauf basierende Empfehlungen, dort oder anderswo gespeichert und bzw. oder der Person zum Beispiel über ein Anzeigeelement der Vorrichtung und bzw. oder über ein Anzeigeelement des mobilen Endgeräts wie zum Beispiel eines Tablets oder eines Smartphones angezeigt und insbesondere visualisiert werden können. Die bioelektrische Impedanzanalyse kann jedoch auch direkt von der Vorrichtung selbst ausgeführt werden, sodass eine Übertragung der Sensorwerte nach außerhalb der Vorrichtung nicht erforderlich ist. Dies kann von einer Steuerungseinheit der Vorrichtung durchgeführt werden, welche vorzugsweise ferner als zentrale Steuerungseinheit die Vorrichtung insgesamt betreibt bzw. steuert.

Um der zu vermessenden Person bzw. um geschultem Personal, welches die zu vermessende Person bei der Verwendung der Vorrichtung unterstützt, die Möglichkeit zu geben, die Vorrichtung an die Größe der Person dahingehend anzupassen, dass die Handelektroden in der vertikalen Richtung für die Person mit den Händen erreichbar sind, ist es aus der DE 10 2010 023 122 A1 bekannt, die Handelektroden in der vertikalen Richtung höhenverstellbar auszubilden. Dies kann jedoch nicht sicherstellen, dass die Person eine definierte Körperhaltung einnimmt, welche zur Erfassung von Sensorwerten für eine bioelektrische Impedanzanalyse geeignet sind. Vielmehr werden gerade viel mehr Einstellmöglichkeiten der Handelektroden der Vorrichtung der DE 10 2010 023 122 A1 zu Verfügung gestellt, welche nicht der vorbestimmten, definierten und bzw. oder reproduzierbaren Körperhaltung der Person entsprechen. Insbesondere kann hierdurch nicht sichergestellt werden, dass die Person bei wiederholter Verwendung der Vorrichtung definierte Körperhaltung reproduzierbar einnimmt. Dies stellt einen schwerwiegenden Störfaktor bei elektrischen Impedanzanalysen dar.

Entsprechend sind die Handelektroden der Vorrichtung daher derart ausgebildet, dass sie von der Person in der vertikalen Richtung nach oben und nach unten zwischen einer ersten, unteren Position und einer zweiten, oberen Position bewegt werden können, indem die Person die Handelektroden in der ersten, unteren Position greift und in die zweite, obere Position bewegt, führt bzw. zieht. Die erste, untere Position stellt einen unteren Endanschlag der Handelektroden dar, sodass die Bewegung der Handelektroden in der vertikalen Richtung durch die erste, untere Position begrenzt ist und die Handelektroden somit aufgrund ihres Eigengewichts und der Schwerkraft von alleine die erste, untere Position einnehmen, solange die Handelektroden von der Person nicht in die zweite, obere Position überführt werden. Entsprechend ruhen die Handelektroden von alleine stets in der ersten, unteren Position.

Bei der zweiten, oberen Position der Handelektroden handelt es sich um eine Positionierung bzw. um eine Lage der Handelektroden entlang der Achse der vertikalen Richtung, welche ungleich der ersten, unteren Position sowie in der vertikalen Richtung nach oben versetzt bzw. beabstandet zu dieser ist. Die Lage bzw. die Höhe der zweiten, oberen Position der Handelektroden ist seitens der Vorrichtung unbestimmt und wird vielmehr durch die Körpergröße der Person bestimmt. Seitens der Vorrichtung findet vorzugsweise lediglich eine Begrenzung der Bewegung der Handelektroden in der vertikalen Richtung nach oben dahingehend statt, dass die Handelektroden nicht versehentlich von der Vorrichtung bzw. von Seitenelementen eines Rahmens der Vorrichtung entfernt bzw. heruntergezogen werden können.

Möchte eine Person nun die Vorrichtung zur Durchführung einer bioelektrischen Impedanzanalyse und gegebenenfalls zur Bestimmung weiterer Gesundheitsparameter verwenden, so findet die Person die Vorrichtung mit den Handelektroden in der ersten, unteren Position ruhend vor. Stellt sich die Person nun mit beiden Füßen auf die beiden Fußelektroden und greift die beiden Handelektroden mit beiden Händen, so ist die erste, untere Position der Handelektroden in der vertikalen Richtung derart weit unten angeordnet, dass jede Person, für welche die Vorrichtung bestimmt ist, eine gebeugte Körperhaltung einnehmen muss, um mit beiden Händen die beiden Handelektroden erreichen und greifen zu können. Hierzu kann die Person in die Knie gehen und bzw. oder den Rücken beugen.

Da eine derart gebeugte Körperhaltung für die Person üblicherweise anstrengend, ungewohnt bzw. unangenehm ist, wird sich die Person intuitiv in eine gerade Körperhaltung aufrichten, sobald die Person die Handelektroden erreicht und mit den Händen gegriffen hat. Hierdurch werden die Handelektroden in die zweite, obere Position überführt, sodass die zweite, obere Position der Handelektroden einfach, direkt und insbesondere intuitiv von der Person eingenommen bzw. erreicht wird. Hierdurch wird erfindungsgemäß erreicht, dass eine definierte bzw. vorbestimmte Position der Handelektroden in Form der zweiten, oberen Position durch die Person selbst, insbesondere durch die Körpergröße sowie durch die Länge der Arme der Person, auf einfache Art und Weise vorgegeben werden kann. Insbesondere sind hierzu weder konkrete Anweisungen an die Person noch die Unterstützung durch geschultes Personal erforderlich, da das Aufrichten aus einer gebeugten Körperhaltung in eine aufrechte Körperhaltung ein intuitives Verhalten der Person darstellt, sobald die Person die Handelektroden mit den Händen gegriffen hat.

Insbesondere kann hierdurch die Reproduzierbarkeit der Körperhaltung der Person bei der Durchführung der Messung gewährleistet werden, da sich üblicherweise die Maße der Person zwischen aufeinanderfolgenden Messungen nicht verändert und hierdurch die zweite, obere Position bei sich wiederholenden Verwendungen der Vorrichtung stets mit sehr hoher Genauigkeit wieder eingenommen bzw. erreicht werden kann. Insbesondere ist es nicht erforderlich, hierzu Einstellungen an der Vorrichtung vorzunehmen und bzw. oder eine Information zu speichern, um die Lage der zweiten, oberen Position der Handelektroden der Person zuordnen und zur Durchführung einer erneuten Messung einstellen zu können.

Die Fußelektroden und bzw. oder Handelektroden bzw. die hierüber erfassten Sensorwerte zur Durchführung einer bioelektrischen Impedanzanalyse können zusätzlich auch zur Erstellung eines Elektrokardiogramms verwendet werden. Dies kann ebenfalls außerhalb der Vorrichtung, wie zuvor beschrieben, oder seitens der Vorrichtung selbst erfolgen, wie weiter unten noch näher beschrieben werden wird. In jedem Fall kann auch ein Elektrokardiogramm und bzw. oder hieraus gewonnene Informationen bzw. Empfehlungen der Person mittels eines Anzeigeelements der Vorrichtung angezeigt werden.

Gemäß einem Aspekt sind die Handelektroden ausgebildet, in der vertikalen Richtung jeweils oder gemeinsam mit einem Höhenverstellelement mit bewegt zu werden, wobei das Höhenverstellelement ausgebildet ist, eine Bewegung der Handelektroden in der vertikalen Richtung nach unten, vorzugsweise mittels wenigstens eines in der vertikalen Richtung ausgerichteten Bewegungsdämpfungselements, zu dämpfen. Mittels eines derartigen Höhenverstellelements kann eine mechanische und in der vertikalen Richtung bewegliche Verbindung zwischen den Handelektroden und den Fußelektroden bzw. weiteren Elementen der Vorrichtung geschaffen werden. Insbesondere können die Handelektroden an einem gemeinsamen Höhenverstellelement oder jeweils an einem separaten Höhenverstellelement angeordnet sein. In jedem Fall kann das Höhenverstellelement bzw. können die Höhenverstellelemente gegenüber einem Rahmen der Vorrichtung in der vertikalen Richtung beweglich sein. Über einen derartigen Rahmen kann gegebenenfalls auch eine feststehende Verbindung mit den beiden Fußelektroden erfolgen.

Dabei eine Dämpfung vorzusehen, welche einer Bewegung des Höhenverstellelements in der vertikalen Richtung nach unten entgegen wirken kann, kann es ermöglichen, dass die Person die Handelektroden bzw. die jeweilige Handelektroden mit der Hand in der zweiten, oberen Position loslassen kann, ohne dass die Handelektroden bzw. das Höhenverstellelement durch seine eigene Gewichtskraft in der vertikalen Richtung unkontrolliert nach unten fällt und in der ersten, unteren Position "hart" in den Endanschlag schlägt. Dies könnte zu unangenehmen Geräuschen für den Benutzer führen und bzw. oder Beschädigungen der Vorrichtung, insbesondere der Handelektroden bzw. des Höhenverstellelements verursachen. Vielmehr eine dämpfende Wirkung bei einer freien Bewegung der Handelektrode bzw. des Höhenverstellelements in der vertikalen Richtung nach unten vorzusehen kann gewährleisten, dass diese Bewegung gedämpft mit einer deutlich geringeren und insbesondere mit einer konstanten Geschwindigkeit und nicht als ein freier Fall erfolgt, wodurch ein "weicher" Kontakt in den Endanschlag in der ersten, unteren Position erreicht werden kann.

Als ein derartiges Dämpfungselement können insbesondere Dämpfer bzw. Puffer verwendet werden, wie sie bei Möbeln zum Beispiel bei Schubladen zum Dämpfen translatorischer Bewegungen eingesetzt werden. Dies kann eine einfache, kostengünstige und bzw. oder platzsparende Umsetzung ermöglichen.

Gemäß einem weiteren Aspekt sind die Handelektroden einander in der Querrichtung, vorzugsweise diametral zur Person, gegenüberliegend seitlich des Paares von Fußelektroden angeordnet. Hierdurch kann eine Körperhaltung der Person bei der Verwendung der Vorrichtung ermöglicht bzw. begünstigt werden, in welcher sich die Person im Wesentlichen innerhalb einer Ebene befindet, welche durch die vertikale Richtung sowie durch die Querrichtung gebildet werden. Dies kann eine für die Person angenehme Körperhaltung darstellen und somit die Verwendung der Vorrichtung begünstigen. Auch kann dies eine vorbestimmte, definierte und bzw. oder intuitiv reproduzierbare Körperhaltung für die Person darstellen. Ferner kann dies eine in der Längsrichtung bzw. in der Tiefe kompakte Ausbildung der Vorrichtung begünstigen und somit den Platzbedarf der Vorrichtung gering halten.

Gemäß einem weiteren Aspekt sind die Handelektroden in der Querrichtung derart beabstandet, so dass die Person in der zweiten, oberen Position eine seitlich abgespreizte Armhaltung einnimmt. Hierdurch kann vermieden werden, dass die Arme der Person bei der Messung mit dem Oberkörper bzw. mit der Hüfte der Person in Kontakt kommen, was eine große Fehlerquelle bei der Durchführung einer Messung für eine bioelektrischen Impedanzanalyse darstellen würde. Durch eine entsprechende Anordnung der Handelektroden in der Querrichtung kann dies sicher sowie intuitiv für die Person umsetzbar vermieden werden.

Gemäß einem weiteren Aspekt weist die erste Handelektrode ein erstes Handelektrodenelement und ein zweites Handelektrodenelement auf, und bzw. oder die zweite Handelektrode weist ein erstes Handelektrodenelement und ein zweites Handelektrodenelement auf, und bzw. oder die erste Fußelektrode weist ein erstes Fußelektrodenelement und ein zweites Fußelektrodenelement auf und bzw. oder die zweite Fußelektrode weist ein erstes Fußelektrodenelement und ein zweites Fußelektrodenelement auf. Mit anderen Worten kann die Vorrichtung statt der vier Elektroden der beiden Handelektroden und der beiden Fußelektroden weitere Elektroden aufweisen, indem die Handelektroden und bzw. oder die Fußelektroden jeweils in voneinander unabhängig für Messungen verwendbare Elektrodenelemente unterteilt sind, welche als Handelektrodenelemente bzw. Fußelektrodenelemente bezeichnet werden können und für sich unabhängig nutzbare Elektroden darstellen können. Hierdurch können bis zu acht unabhängige Elektroden geschaffen werden, um die Anzahl der Messpunkte an den Händen und bzw. oder an den Füßen der Person für Messungen entsprechend zu erhöhen. Beispielsweise kann auf diese Art und Weise mit acht Elektroden eine oktopolare bioelektrische Impedanzanalyse durchgeführt werden. Dies kann die Möglichkeiten zur Messung sowie zur Auswertung von Gesundheitsparametern der Person entsprechend erhöhen und bzw. oder die Qualität der Messungen verbessern.

Gemäß einem weiteren Aspekt sind das erste Handelektrodenelement und das zweite Handelektrodenelement der ersten Handelektrode einander in der vertikalen Richtung, vorzugsweise diametral, gegenüberliegend, vorzugsweise an einem ersten höhenverstellbaren Handelement, angeordnet, und bzw. oder das erste Handelektrodenelement und das zweite Handelektrodenelement der zweiten Handelektrode sind einander in der vertikalen Richtung, vorzugsweise diametral, gegenüberliegend, vorzugsweise an einem zweiten höhenverstellbaren Handelement, angeordnet. Hierdurch können die Handelektrodenelemente derart, insbesondere an einem Handelement, angeordnet werden, dass das erste Handelektrodenelement mit einer Handinnenfläche der Person und das zweite Handelektrodenelement mit der Innenseite der Finger der Person kontaktiert werden kann, oder umgekehrt. Dies kann zwei voneinander unabhängige elektrische Kontaktierung an derselben Hand der Person ermöglichen.

Gemäß einem weiteren Aspekt ist die Steuerungseinheit der Vorrichtung, ferner ausgebildet, ein Elektrokardiogramm zu erstellen. Mit anderen Worten können die Handelektroden und bzw. oder die Fußelektroden bzw. die dort erfassten Sensorwerte zusätzlich zur bioelektrischen Impedanzanalyse auch zur Erstellung eines Elektrokardiogramms verwendet werden, wie bereits zuvor erwähnt. Dies kann die Auswertemöglichkeiten erhöhen. Dieser Auswertung seitens der Steuerungseinheit der Vorrichtung vorzunehmen kann eine direkte Umsetzung ermöglichen. Vorzugsweise kann das erstellte Elektrokardiogramm und bzw. oder hieraus resultierende weitere Informationen bzw. Empfehlungen durch ein Anzeigeelement der Vorrichtung der Person angezeigt werden.

Gemäß einem weiteren Aspekt weist die Vorrichtung wenigstens einen optischen Sensor auf, welcher ausgebildet ist, Sensorwerte zur Durchführung einer Photoplethysmographie und bzw. oder einer Pulsoxymetrie zu erfassen. Ein derartiger optischer Sensor kann sowohl eine Signallichtquelle zum Beispiel in Form einer monochromatischen Leuchtdiode (LED) oder mehrerer Leuchtdioden mit verschiedenen charakteristischen Wellenlängen des abgestrahlten Lichts, vorzugsweise rot und infrarot strachlende LEDs, und einen Signallichtsensor zum Beispiel in Form einer Fotodiode oder einer Laserdiode aufweisen. Hierdurch können die Möglichkeiten der Vorrichtung um die Photoplethysmographie und bzw. oder um die Pulsoxymetrie erweitert werden.

Der optische Sensor kann hierzu an einer geeigneten Stelle und insbesondere derart angeordnet werden, dass der optische Sensor gleichzeitig bzw. parallel zum Erfassen der Sensorwerte zur Durchführung der bioelektrischen Impedanzanalyse verwendet werden kann. Hierzu kann der optische Sensor derart angeordnet werden, dass die Messwerte erfasst werden können, wenn die Person die Fußelektroden und bzw. oder die Handelektroden wie zuvor beschrieben verwendet, sodass die Person keine zusätzlichen Handlungen hierfür ausführen muss. Hierzu kann der optische Sensor insbesondere mit einer Handelektrode oder mit einer Fußelektrode kombiniert angeordnet werden.

Die Sensorwerte des optischen Sensors können ebenfalls außerhalb der Vorrichtung, wie zuvor beschrieben, oder seitens der Steuerungseinheit der Vorrichtung zur Durchführung einer Photoplethysmographie und bzw. oder einer Pulsoxymetrie verwendet werden. In jedem Fall kann auch eine Photoplethysmographie und bzw. oder eine Pulsoxymetrie und bzw. oder hieraus gewonnene Informationen bzw. Empfehlungen der Person mittels eines Anzeigeelements der Vorrichtung angezeigt werden.

Gemäß einem weiteren Aspekt weist die Vorrichtung einen ersten optischen Handsensor, welcher an einem ersten höhenverstellbaren Handelements, vorzugsweise in der vertikalen Richtung zwischen einem ersten Handelektrodenelement und einem zweiten Handelektrodenelement der ersten Handelektrode und bzw. oder in der Querrichtung zur Person hinzeigend, angeordnet und ausgebildet ist, zur Durchführung einer Photoplethysmographie und bzw. oder einer Pulsoxymetrie verwendet zu werden, und bzw. oder einen zweiten optischen Handsensor, welcher an einem zweiten höhenverstellbaren Handelements, vorzugsweise in der vertikalen Richtung zwischen einem ersten Handelektrodenelement und einem zweiten Handelektrodenelement der zweiten Handelektrode und bzw. oder in der Querrichtung zur Person hinzeigend, angeordnet und ausgebildet ist, zur Durchführung einer Photoplethysmographie und bzw. oder einer Pulsoxymetrie verwendet zu werden, und bzw. oder einen ersten optischen Fußsensor, welcher vorzugsweise innerhalb der ersten Fußelektrode, besonders vorzugsweise innerhalb des ersten Fußelektrodenelements oder innerhalb des zweiten Fußelektrodenelements der ersten Fußelektrode, angeordnet und ausgebildet ist, zur Durchführung einer Photoplethysmographie und bzw. oder einer Pulsoxymetrie verwendet zu werden, und bzw. oder einen zweiten optischen Fußsensor, welcher vorzugsweise innerhalb der zweiten Fußelektrode, besonders vorzugsweise innerhalb des ersten Fußelektrodenelements oder innerhalb des zweiten Fußelektrodenelements der zweiten Fußelektrode, angeordnet und ausgebildet ist, zur Durchführung einer Photoplethysmographie und bzw. oder einer Pulsoxymetrie verwendet zu werden.

Hierdurch können verschiedene konkrete Anordnungen derartiger optischer Sensoren geschaffen werden, um Messungen zur Durchführung einer Photoplethysmographie und bzw. oder einer Pulsoxymetrie vornehmen zu können, wie zuvor beschrieben. Diese Anordnungen können insbesondere geeignet sein, um diese Messungen parallel und zeitgleich zur Verwendung der Handelektroden und bzw. oder der Fußelektroden durchführen zu können.

Hierbei alle vier optischen Sensoren, d.h. bei optische Handsensoren und beide optische Fußsensoren, vorzusehen bzw. zu verwenden kann dahingehend vorteilhaft sein, dass auf diese Art und Weise eine Multi-site Photoplethysmography (MPPG) durchgeführt werden kann. Dabei können aus den Laufzeitunterschieden der Pulswellen zu den vier Messpunkten potentiell weitere Parameter siehe zum Beispiel Blutdruck und bzw. oder Gefäßgesundheit ermittelt werden.

Vorzugsweise ist der erste optische Handsensor innerhalb einer Fingermulde des ersten höhenverstellbaren Handelements angeordnet und bzw. oder ist vorzugsweise der zweite optische Handsensor innerhalb einer Fingermulde des zweiten höhenverstellbaren Handelements angeordnet. Mit anderen Worten weist wenigstens ein höhenverstellbares Handelement eine Vertiefung bzw. einen Rücksprung gegenüber der übrigen Oberfläche auf, welche muldenförmig ausgebildet ist und abschnittsweise einen Finger der Hand der Person aufnehmen kann. Hierdurch kann ein möglichst lichtdichter Abschluss der Fingermulde gegenüber der Umgebung durch den Finger der Hand der Person erfolgen, sodass störendes Fremdlicht von dem optischen Sensor möglichst ferngehalten werden kann. Dies kann die Qualität der sensorischen Erfassung der Messwerte verbessern bzw. gewährleisten.

Gemäß einem weiteren Aspekt weist die Vorrichtung einen Rahmen mit einerr Bodenelement, welches die Fußelektroden aufweist, ein erstes Seitenelement, welches die erste Handelektrode, vorzugsweise ein erstes höhenverstellbares Handelement mit der erste Handelektrode, in der vertikalen Richtung zwischen der ersten, unteren Position und der zweiten, oberen Position höhenverstellbar aufweist, und ein zweites Seitenelement auf, welches die zweite Handelektrode, vorzugsweise ein zweites höhenverstellbares Handelement mit der zweiten Handelektrode, in der vertikalen Richtung zwischen der ersten, unteren Position und der zweiten, oberen Position höhenverstellbar aufweist. Ein derartiger Rahmen kann auch als Gestell oder als Halterung bezeichnet werden. In jedem Fall kann hierdurch eine mechanisch stabile Anordnung der einzelnen Elemente der Vorrichtung zueinander erreicht werden. Auch kann hierdurch eine räumlich definierte Anordnung der einzelnen Elemente der Vorrichtung zueinander erfolgen. Insbesondere kann die Beweglichkeit in der vertikalen Richtung der Handelektroden bzw. deren höhenverstellbarer Handelemente hierdurch ermöglicht werden. Vorzugsweise können dabei das Bodenelement und die beiden Seitenelemente U-förmig angeordnet sein, was die Umsetzung der entsprechenden Funktionen auf einfache und bzw. oder platzsparende Art und Weise ermöglichen kann. Die Seitenelemente können auch einer, vorzugsweise in der vertikalen Richtung höhenverstellbaren, Anordnung eines Anzeigeelements, eines Bedienelements und bzw. oder eines Anzeige-/Bedienelements der Vorrichtung dienen.

Gemäß einem weiteren Aspekt weist der Rahmen ein Dachelement auf, wobei das Bodenelement, das erste Seitenelement, das zweite Seitenelement und das Dachelement eine zumindest im Wesentlichen rechteckige Form bilden, welche ausgebildet ist, die Person in der aufrechten Körperhaltung in sich aufzunehmen. Hierdurch kann eine in einer rechteckigen Umfangsrichtung geschlossene Anordnung der Elemente des Rahmens erreicht werden, was die Stabilität des Rahmens bzw. der Vorrichtung verbessern kann.

Gemäß einem weiteren Aspekt weist die Vorrichtung einen Größenmessungssensor auf, welcher in der vertikalen Richtung derart oberhalb der Fußelektroden angeordnet und zu den Fußelektroden hin ausgerichtet ist, so dass ein Sensorwert, welcher einen Abstand zur Person repräsentiert, erfasst werden kann. Hierdurch kann sensorisch die Größe der Person parallel zu den zuvor beschriebenen Messungen vorgenommen werden, sodass die Größe der Person als weiterer Parameter bzw. als weiterer Gesundheitsparameter erhalten werden kann. Dies kann die hieraus resultierenden Möglichkeiten zur Auswertung des Gesundheitszustands der Person erhöhen.

Die Sensorwerte des Größenmessungssensors können ebenfalls außerhalb der Vorrichtung, wie zuvor beschrieben, oder seitens der Steuerungseinheit der Vorrichtung zur Bestimmung der Größe der Person verwendet werden. In jedem Fall kann auch die Größe der Person und bzw. oder hieraus gewonnene Informationen bzw. Empfehlungen der Person mittels eines Anzeigeelements der Vorrichtung angezeigt werden.

Gemäß einem weiteren Aspekt weist die Vorrichtung eine Waageeinrichtung auf, welche ausgebildet ist, die Person auf sich aufzunehmen und einen das Gewicht der Person repräsentierenden Sensorwert zu erfassen. Die Waageeinrichtung kann auch als Wiegeeinrichtung bezeichnet werden. Hierdurch kann sensorisch das Gewicht der Person parallel zu den zuvor beschriebenen Messungen vorgenommen werden, sodass das Gewicht der Person als weiterer Parameter bzw. als weiterer Gesundheitsparameter erhalten werden kann. Dies kann die hieraus resultierenden Möglichkeiten zur Auswertung des Gesundheitszustands der Person erhöhen.

Die Sensorwerte der Waageeinrichtung können ebenfalls außerhalb der Vorrichtung, wie zuvor beschrieben, oder seitens der Steuerungseinheit der Vorrichtung zur Bestimmung des Gewichts der Person verwendet werden. In jedem Fall kann auch das Gewicht der Person und bzw. oder hieraus gewonnene Informationen bzw. Empfehlungen der Person mittels eines Anzeigeelements der Vorrichtung angezeigt werden.

Gemäß einem weiteren Aspekt ist die Waageeinrichtung in der vertikalen Richtung unterhalb des Paares von Fußelektroden angeordnet. Dies kann eine kompakte Anordnung der Waageeinrichtung ermöglichen. Insbesondere kann hierdurch ermöglicht werden, gleichzeitig und parallel die Fußelektroden und die Waageeinrichtung zu nutzen und entsprechende Messungen zeitgleich durchzuführen. Dies kann insbesondere einen zusätzlichen Aufwand für die Person zur Nutzung der Waageeinrichtung vermeiden.

Gemäß einem weiteren Aspekt weist die Vorrichtung ein Anzeigeelement oder ein Anzeige-/Bedienelement, welches ausgebildet ist, der Person wenigstens einen mittels der bioelektrischen Impedanzanalyse bestimmten Gesundheitsparameters der Person anzuzeigen, und bzw. oder ein Bedienelement oder ein Anzeige-/Bedienelement auf, welches ausgebildet ist, wenigstens eine Eingabe der Person zu erfassen. Das Anzeige-/Bedienelement kann insbesondere als Touchscreen umgesetzt werden. Das Anzeigeelement, das Bedienelement oder das kombinierte Anzeige-/Bedienelement kann höhenverstellbar oder feststehend, insbesondere an einem Rahmen der Vorrichtung angeordnet sein. Die Höhenverstellbarkeit kann es ermöglichen, eine für die jeweiligen Person komfortable Höhe in der vertikalen Richtung einzunehmen, was die Bedienung und bzw. oder die Ansicht für die Person erleichtern bzw. verbessern kann. Die feste Anordnung kann den konstruktiven Aufwand sowie die Kosten der Umsetzung geringer halten. In jedem Fall können seitens der Vorrichtung Möglichkeiten geschaffen werden, dass Informationen der Person angezeigt und bzw. oder Eingaben von der Person an die Vorrichtung getätigt werden können. Zusätzlich oder alternativ können auch hieraus gewonnene Informationen bzw. Empfehlungen der Person angezeigt werden.

Insbesondere kann das Anzeige-/Bedienelement derart verwendet werden, dass sich eine Person als Benutzer der Vorrichtung für eine Selbstvermessung anmelden bzw. identifizieren und hierfür Bedieneingaben tätigen kann. Eine derartige Registrierung mittels Personendaten des Nutzers kann alternativ auch mittels einer App eines mobilen Endgeräts erfolgen. Über das Anzeige-/Bedienelement kann die identifizierte bzw. registrierte Person auch vor Kontraindikationen gewarnt werden, bevor Messungen vorgenommen werden.

In jedem Fall können dann einzelne, mehrere bzw. alle zuvor beschriebenen Messungen mittels der Vorrichtung durchgeführt werden. Hierbei kann die Person durch eine schrittweise Anleitung der durchzuführenden Maßnahmen der Person wie z.B. das Greifen der Handelektroden, das anschließende Aufrichten, das Abwarten der Durchführung der Messungen über einen Zeitraum sowie das Loslassen der Handelektroden geleitet werden, was über Ausgaben in Textform und bzw. oder in graphischer Darstellung auf dem Anzeige-/Bedienelement erfolgen kann. Insbesondere das Einnehmen einer korrekten Messposition kann durch audio-visuelle Anleitung über das Anzeige-/Bedienelement unterstützt werden.

Nach erfolgter Durchführung der Messung bzw. Messungen können die ausgewerteten Messergebnisse und bzw. oder hieraus resultierende Informationen bzw. Empfehlungen der Person auf dem Anzeige-/Bedienelement angezeigt werden. Zusätzlich oder alternativ können die Messergebnisse, Informationen bzw. Empfehlungen auch auf eine App eines mobilen Endgerätes der Person übertragen werden. Dies kann auch umfassen, dass am Ende der Messung die Person nur mit der App einen QR Code vom Anzeige-/Bedienelement abscannen muss. Zusätzlich oder alternativ können die Messergebnisse, Informationen bzw. Empfehlungen der Person auch per E-Mail zugesandt werden.

Zwei Ausführungsbeispiele und weitere Vorteile werden nachstehend im Zusammenhang mit den folgenden Figuren rein schematisch dargestellt und näher erläutert. Darin zeigt:
- Figur 1: eine perspektivische schematische Darstellung einer Vorrichtung gemäß eines ersten Ausführungsbeispiels;
- Figur 2: eine perspektivische Detailansicht des unteren Bereichs der Vorrichtung gemäß des ersten Ausführungsbeispiels;
- Figur 3: eine perspektivische Detailansicht eines ersten, linken höhenverstellbaren Handelements der Vorrichtung gemäß des ersten Ausführungsbeispiels;
- Figur 4: eine perspektivische Detailansicht eines zweiten, rechten höhenverstellbaren Handelements der Vorrichtung gemäß des ersten Ausführungsbeispiels;

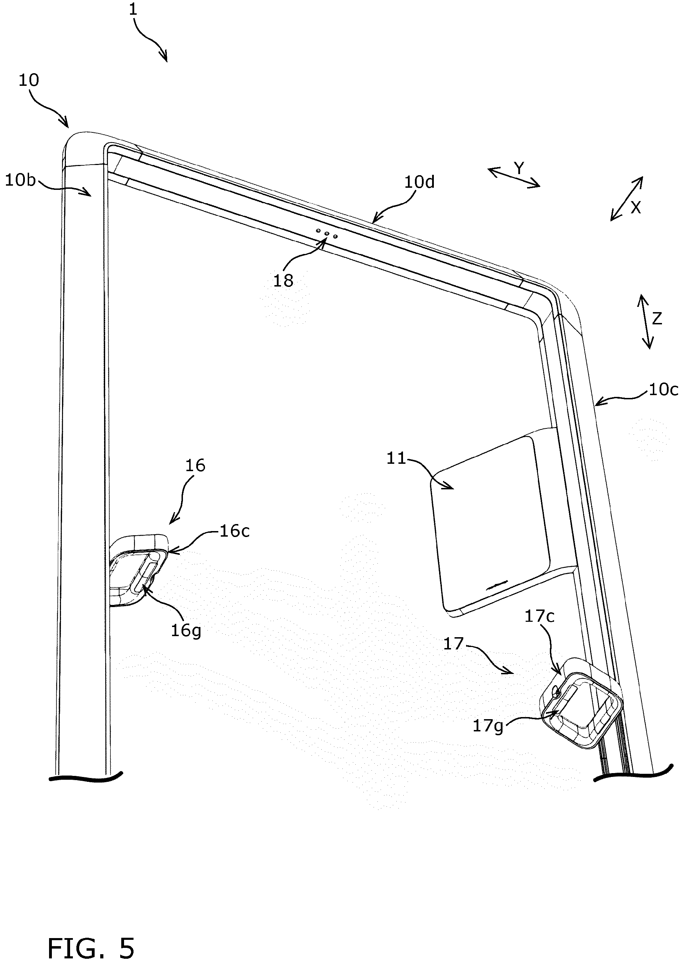

- Figur 5: eine perspektivische Detailansicht des oberen Bereichs der Vorrichtung gemäß des ersten Ausführungsbeispiels;
- Figur 6: eine Seitenansicht auf die Vorrichtung gemäß des ersten Ausführungsbeispiels mit einer Person in gebeugter Körperhaltung und mit den höhenverstellbaren Handelementen in einer ersten, unteren Position;
- Figur 7: eine Seitenansicht auf die Vorrichtung gemäß des ersten Ausführungsbeispiels mit einer Person in aufrechter Körperhaltung und mit den höhenverstellbaren Handelementen in einer zweiten, oberen Position; und
- Figur 8: die Darstellung der Figur 2 gemäß eines zweiten Ausführungsbeispiels.

Die o.g. Figuren werden in kartesischen Koordinaten betrachtet. Es erstreckt sich eine Längsrichtung X, welche auch als Tiefe X oder als Länge X bezeichnet werden kann. Senkrecht zur Längsrichtung X erstreckt sich eine Querrichtung Y, welche auch als Breite Y bezeichnet werden kann. Senkrecht sowohl zur Längsrichtung X als auch zur Querrichtung Y erstreckt sich eine vertikale Richtung Z, welche auch als Höhe Z bezeichnet werden kann und der Richtung der Schwerkraft entspricht. Die Längsrichtung X und die Querrichtung Y bilden gemeinsam die Horizontale X, Y, welche auch als horizontale Ebene X, Y bezeichnet werden kann.

Eine Vorrichtung 1 dient der Bestimmung wenigstens eines Gesundheitsparameters einer Person 2. Die Person 2 kann daher auch als zu vermessene Person 2 bezeichnet werden und stellt ein biologisches Subjekt 2 dar. Die Vorrichtung 1 kann auch als multifunktionale Messvorrichtung 1 bezeichnet werden.

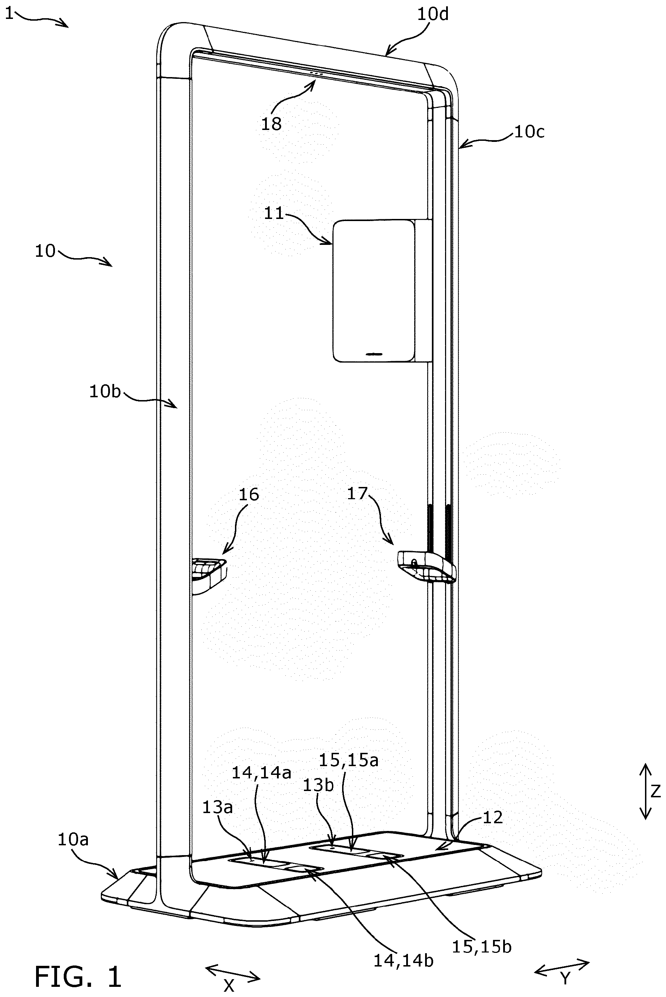

Die Vorrichtung 1 weist einen Rahmen 10 auf, welcher auch als Rahmenelement 10 bezeichnet werden kann, siehe zum Beispiel Figur 1. Der Rahmen 10 weist ein Bodenelement 10a auf, welches im Wesentlichen flächig in der Horizontalen X, Y ausgebildet ist und bei bestimmungsgemäßer Verwendung der Vorrichtung 1 auf einem Untergrund wie zum Beispiel auf einem Fußboden angeordnet wird. In der Querrichtung Y ist links am Rand des Bodenelements 10a des Rahmens 10 ein erstes, linkes Seitenelement 10b des Rahmens 10 feststehend angeordnet, welches sich senkrecht in der vertikalen Richtung Z nach oben erstreckt. In der Querrichtung Y symmetrisch gegenüberliegend ist rechts am Rand des Bodenelements 10a des Rahmens 10 ein baugleiches zweites, rechtes Seitenelement 10c des Rahmens 10 feststehend angeordnet, welches sich parallel zum ersten, linken Seitenelement 10b des Rahmens 10 senkrecht in der vertikalen Richtung Z nach oben erstreckt. An ihren oberen Enden sind die beiden Seitenelemente 10b, 10c des Rahmens 10 in der Querrichtung Y durch ein waagerecht verlaufendes Dachelement 10d des Rahmens 10 feststehend miteinander verbunden. Hierdurch bilden das Bodenelement 10a, die beiden Seitenelemente 10b, 10c sowie das Dachelement 10d den Rahmen 10 mit einer rechteckigen Kontur, welche im Wesentlichen in der Ebene der vertikalen Richtung Z und der Querrichtung Y verläuft.

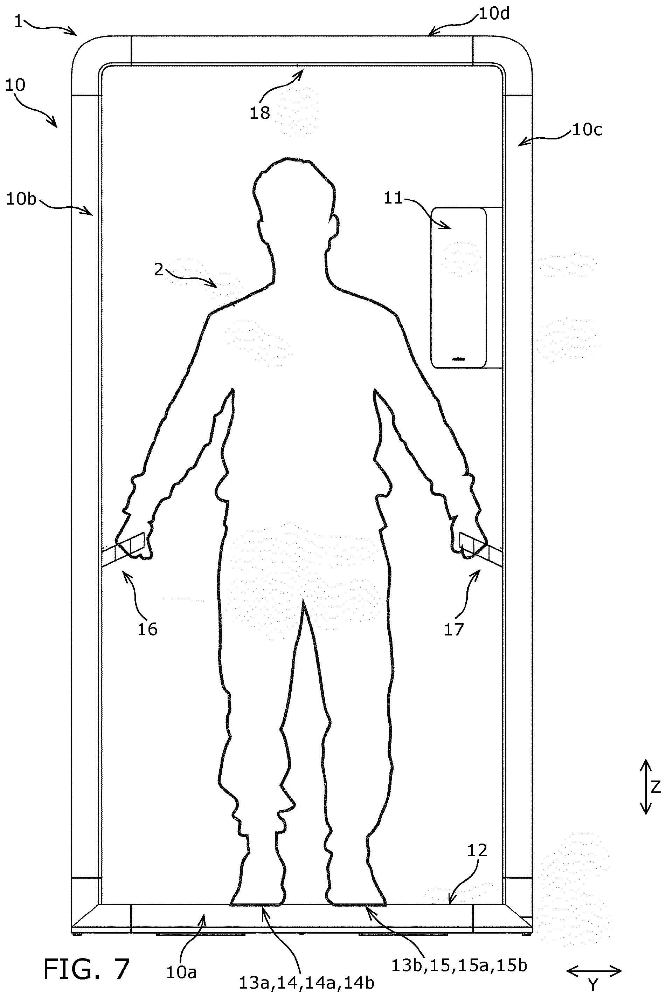

Der Platz innerhalb des Rahmens 10 ist dabei in der vertikalen Richtung Z derart bemessen, dass auch sehr große Personen 2 auf dem Bodenelement 10a des Rahmens 10 aufrecht stehen können, ohne das Dachelement 10d des Rahmens 10 zu berühren, siehe zum Beispiel Figur 7. In der Querrichtung Y ist der Platz innerhalb des Rahmens 10 derart bemessen, dass auch sehr breite Personen 2 auf dem Bodenelement 10a des Rahmens 10 stehen können, ohne die beiden Seitenelemente 10b, 10c des Rahmens 10 mit ihren Armen zu berühren. Ferner ist der Abstand zwischen den beiden Seitenelementen 10b, 10c des Rahmens 10 in der Querrichtung Y derart bemessen, dass die auf dem Bodenelement 10a des Rahmens 10 stehende Person 2 die beiden Seitenelemente 10b, 10c des Rahmens 10 nur mit abgewinkelten Armen erreichen bzw. berühren kann.

In einer Höhe in der vertikalen Richtung Z, welche etwa auf Höhe des Gesichts der aufrechtstehenden Person 2 liegt, ist am zweiten, rechten Seitenelement 10c des Rahmens 10 ein Anzeige-/Bedienelement 11 in Form eines Touchdisplays 11 angeordnet, siehe zum Beispiel Figur 1, welches in der vertikalen Richtung Z entlang des zweiten, rechten Seitenelements 10c des Rahmens 10 ausreichend höhenverstellbar ist, um an die Blickhöhe unterschiedlich großer Personen 2 angepasst zu werden. Das Anzeige-/Bedienelement 11 kann der Person 2 während der Benutzung der Vorrichtung 1 Informationen anzeigen und somit als Anzeigeelement 11 verwendet werden. Über das Anzeige-/Bedienelement 11 kann die Person 2 während der Benutzung der Vorrichtung 1 als Bedienelement 11 Eingaben an die Vorrichtung 1 tätigen, um diese zu bedienen.

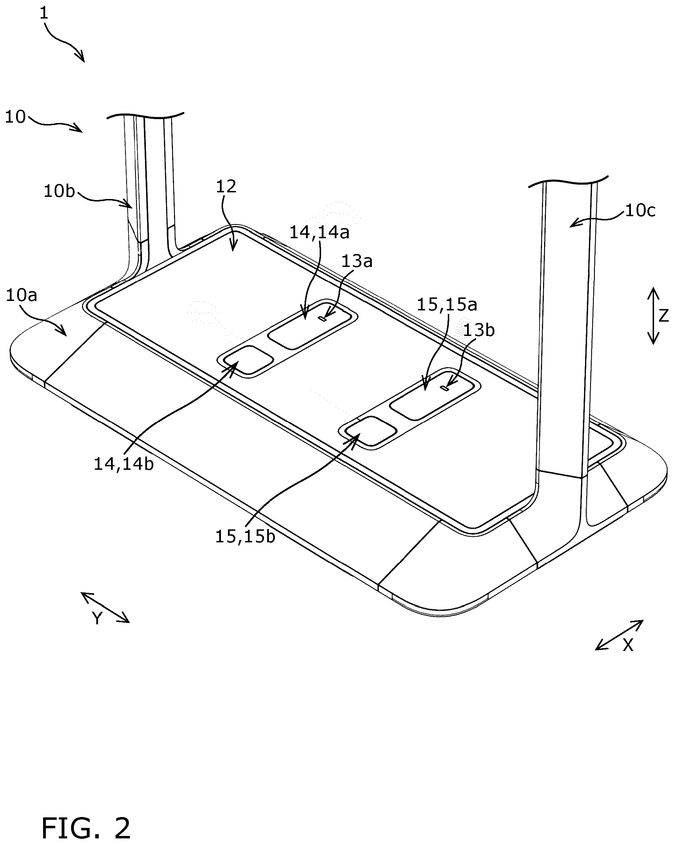

Das Bodenelement 10a des Rahmens 10 weist eine Waageeinrichtung 12 auf, d. h. eine Vorrichtung zum Wiegen 12, siehe zum Beispiel Figur 2, welche innerhalb der Horizontalen X, Y mittig im Bodenelement 10a des Rahmens 10 derart angeordnet ist, dass die Waageeinrichtung 12 von der Person 2 benutzt wird, wenn die Person 2 mittig auf dem Bodenelement 10a des Rahmens 10 steht. Die Waageeinrichtung 12 wird dabei von einem Rand (nicht bezeichnet) des Bodenelements 10a des Rahmens 10 umschlossen. Die Waageeinrichtung 12 kann einen Sensorwert erfassen, welcher ein Gewicht repräsentiert, welches als Gewicht der Person 2 in der vertikalen Richtung Z von oben auf die Waageeinrichtung 12 ausgeübt wird, wenn die Person 2 auf der Waageeinrichtung 12 steht. Der Sensorwert der Waageeinrichtung 12 kann von einer Steuerungseinheit (nicht dargestellt) der Vorrichtung 1 ausgewertet und in einen entsprechenden Wert gewandelt werden, welcher dem Gewicht der Person 2 entspricht. Die Information des Gewichts der Person 2 kann von dem Anzeigeelement 11 der Person 2 angezeigt und bzw. oder weiterverarbeitet und insbesondere mit anderen Gesundheitsparametern der Person 2 kombiniert ausgewertet werden.

Das Bodenelement 10a des Rahmens 10 weist ferner auf der Waageeinrichtung 12 drauf eine erste, linke Fußelektrode 14 und eine zweite, rechte Fußelektrode 15 auf, siehe zum Beispiel Figur 2. Die erste, linke Fußelektrode 14 ist in ein erstes, vorderes Fußelektronenelement 14a für den Ballen des Fußes der Person 2 und in ein zweites, hinteres Fußelektrodenelement 14b für die Verse des Fußes der Person 2 unterteilt, wobei beide Fußelektrodenelemente 14a, 14b voneinander unabhängig verwendbare Elektroden darstellen. Entsprechend weist auch die zweite, rechte Fußelektrode 15 ein erstes, vorderes Fußelektrodenelement 15a und ein zweites, hinteres Fußelektrodenelement 15b auf. Die beiden Fußelektroden 14, 15 bzw. ihre Fußelektrodenelemente 14a, 14b, 15a, 15b sind derart zueinander sowie derart gegenüber den übrigen Elementen der Vorrichtung 1 angeordnet, dass die Person 2 mit Blickrichtung in der Längsrichtung X mit beiden Füßen in der vertikalen Richtung Z von oben auf den Fußelektroden 14, 15 stehen kann.

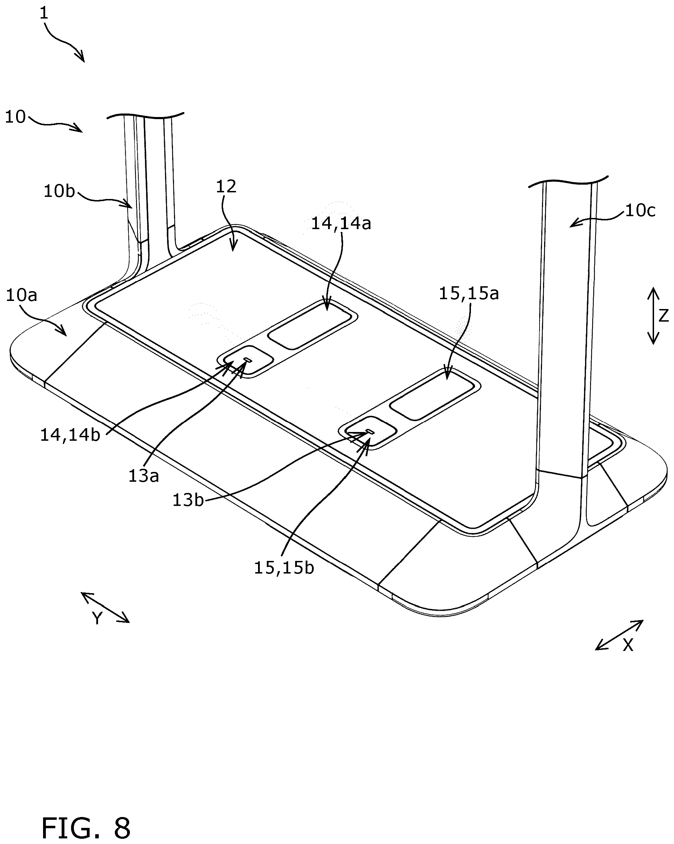

Gemäß dem ersten Ausführungsbeispiel der Vorrichtung 1 ist innerhalb des ersten, vorderen Fußelektrodenelements 14a der ersten, linken Fußelektrode 14 ein erster, linker optischer Fußsensor 13a angeordnet und in der vertikalen Richtung Z nach oben hin ausgerichtet, siehe zum Beispiel Figur 2. Ebenso ist innerhalb des ersten, vorderen Fußelektrodenelements 15a der zweiten, rechten Fußelektrode 15 ein zweiter, rechter optischer Fußsensor 13b angeordnet und in der vertikalen Richtung Z nach oben hin ausgerichtet. Gemäß dem zweiten Ausführungsbeispiel der Vorrichtung 1 gemäß der Figur 8 sind die beiden optischen Fußsensoren 13a, 13b innerhalb der beiden zweiten, hinteren Fußelektrodenelemente 14b, 15b der beiden Fußelektroden 14, 15 angeordnet. In jedem Fall weisen beide Fußsensoren 13a, 13b jeweils eine Kombination aus einer Signallichtquelle (nicht dargestellt) in Form einer monochromatischen Leuchtdiode oder einer oder mehrerer Leuchtdioden verschiedener Wellenlängen und aus einem Signallichtsensor (nicht dargestellt) in Form einer entsprechenden Fotodiode oder Laserdiode auf. Mittels der Fußsensoren 13a, 13b können sensorische Messungen zur Durchführung einer Photoplethysmographie ausgeführt werden, während die Person 2 mit den Füßen auf den Fußelektroden 14, 15 steht.

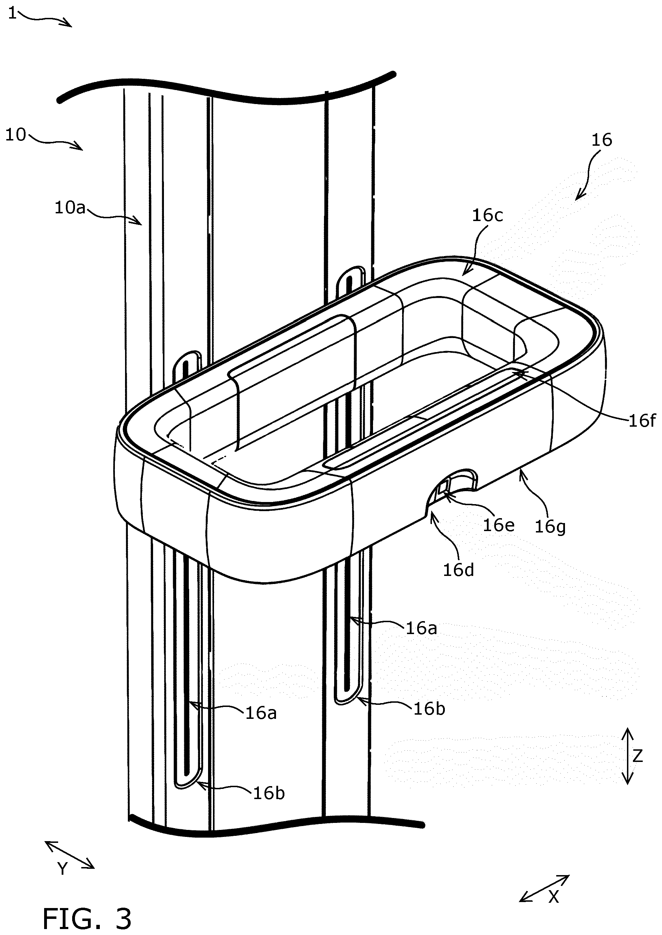

An dem ersten, linken Seitenelement 10b des Rahmens 10 ist an der Innenseite, d. h. dem zweiten, rechten Seitenelement 10c des Rahmens 10 zugewandt, ein erstes, linkes höhenverstellbares Handelement 16 angeordnet, welches in der vertikalen Richtung Z bewegt werden kann, siehe zum Beispiel Figur 3. Das erste, linke Seitenelement 10b des Rahmens 10 weist ein Paar von sich länglich in der vertikalen Richtung Z erstreckenden Durchgangsöffnungen 16b auf. Innerhalb des ersten, linken Seitenelements 10b des Rahmens 10 ist ein Höhenverstellelement 16a bzw. eine Höhenverstellung 16a angeordnet, welche durch die Durchgangsöffnungen 16b des Seitenelements 10c des Rahmens 10 hindurch mit einem Griffelement 16c verbunden ist, welches auch als Griffstück 16c oder als Handgriff 16c bezeichnet werden kann. Das Höhenverstellelement 16a weist einen feststehenden Teil auf, welcher feststehend mit dem Seitenelement 10c des Rahmens 10 verbunden ist, siehe zum Beispiel Figur 3. Das Höhenverstellelement 16a weist einen beweglichen Teil (nicht dargestellt) auf, welcher feststehend mit dem Griffelement 16c verbunden und in der vertikalen Richtung Z gegenüber dem feststehenden Teil des Höhenverstellelements 16a beweglich ist. Hierdurch kann das Griffelement 16c in der vertikalen Richtung Z gegenüber dem Seitenelement 10c des Rahmens 10 bewegt werden. Das Maß der Bewegung in der vertikalen Richtung Z wird dabei durch die längliche Erstreckung der Durchgangsöffnung 16b des Seitenelements 10c des Rahmens 10 begrenzt.

Das Höhenverstellelement 16a weist ein Dämpfungselement auf, wie es von Möbeln zur Dämpfung der translatorischen Bewegung zum Beispiel einer Schublade bekannt ist, um die Bewegung des ersten, linken höhenverstellbaren Handelements 16 bzw. dessen Griffelements 16c in der vertikalen Richtung Z nach unten zu dämpfen und hierbei eine konstante Geschwindigkeit zu erreichen, welche ein sanftes Aufsetzen des ersten, linken höhenverstellbaren Handelements 16 bzw. dessen Griffelements 16c am unteren Ende der Durchgangsöffnung 16b des Seitenelements 10c des Rahmens 10 ermöglicht. Am unteren Ende der Durchgangsöffnung 16b des Seitenelements 10c des Rahmens 10 angekommen nimmt das erste, linke höhenverstellbare Handelement 16 bzw. dessen Griffelement 16c eine erste, untere Position ein, in welcher das erste, linke höhenverstellbare Handelement 16 bzw. dessen Griffelement 16c ruht.

Das Griffelement 16c des ersten, linken höhenverstellbaren Handelements 16 ist in der Horizontalen X, Y flächig sowie im Wesentlichen rechteckig ausgebildet und weist mittig eine Durchgangsöffnung (nicht bezeichnet) auf, durch welche hindurch die Person 2 die linke Hand in der vertikalen Richtung Z von oben nach unten hindurchstecken und das Griffelement 16c mit den Fingern dort umgreifen kann, wo das Griffelement 16c dem Seitenelement 10c des Rahmens 10 abgewandt ist, siehe zum Beispiel Figur 3.

In diesem Bereich, welcher als Griffbereich bezeichnet werden kann, ist in der vertikalen Richtung Z an der Oberseite (nicht bezeichnet) des Griffelements 16c ein erstes, oberes Handelektrodenelement 16f sich im Wesentlichen in der Längsrichtung X erstreckend ausgebildet. In der vertikalen Richtung Z gegenüberliegend ist in diesem Bereich an der Unterseite (nicht bezeichnet) des Griffelements 16c dem ersten, oberen Handelektrodenelement 16f genau bzw. diametral gegenüberliegend ein zweites, unteres Handelektrodenelement 16g sich im Wesentlichen in der Längsrichtung X erstreckend angeordnet. Die beiden Handelektrodenelemente 16f, 16g stellen unabhängig voneinander nutzbare Elektroden dar, welche gemeinsam als erste, linke Handelektrode 16f, 16g bezeichnet werden können.

Die erste, linke Handelektrode 16f, 16g wird an ihrem ersten, oberen Handelektrodenelement 16f von der linken Hand der Person 2 mit der Handfläche und an ihrem zweiten, unteren Handelektrodenelement 16g von der linken Hand der Person 2 mit den Innenseiten der Finger elektrisch kontaktiert, wenn die Person 2 das Griffelement 16c des ersten, linken höhenverstellbaren Handelements 16 wie zuvor beschrieben umgreift. Hierdurch kann ein elektrisch leitfähiger Kontakt zwischen dem Griffelement 16c und der linken Hand der Person 2 an zwei Stellen hergestellt werden, um Messungen zur Bestimmung von Gesundheitsparametern durchzuführen, welche elektrische Spannungen bzw. elektrische Spannungsänderungen nutzen. Hierzu gehören zum einen die bioelektrische Impedanzanalyse und zum anderen die Elektrokardiografie.

Das Griffelement 16c des ersten, linken höhenverstellbaren Handelements 16 weist ferner im Griffbereich dem ersten, linken Seitenelement 10c des Rahmens 10 abgewandt eine Fingermulde 16d auf, in welcher ein erster, linker optischer Handsensor 16e angeordnet ist. Der erste, linke optische Handsensor 16b ist in der Querrichtung Y dem ersten, linken Seitenelement 10c des Rahmens 10 abgewandt bzw. zur Person 2 hin zeigend ausgerichtet. Wird nun das Griffelement 16c wie zuvor beschrieben von der linken Hand der Person 2 im Griffbereich umgriffen, so kann die Person 2 den Mittelfinger der linken Hand in die Fingermulde 16d legen. Hierdurch wird zum einen der Mittelfinger derart nahe am ersten, linken optischen Handsensor 16e bzw. auf dem ersten, linken optischen Handsensor 16e drauf positioniert, so dass Messungen zur Durchführung einer Photoplethysmographie ausgeführt werden können, während die Person 2 das Griffelement 16c des ersten, linken höhenverstellbaren Handelements 16 mit der linken Hand umgreift. Zum anderen wird dank der Fingermulde 16d, dessen Formgebung etwa der Kontur eines Mittelfingers entspricht, störendes Fremdlicht von dem ersten, linken optischen Handsensor 16e ferngehalten.

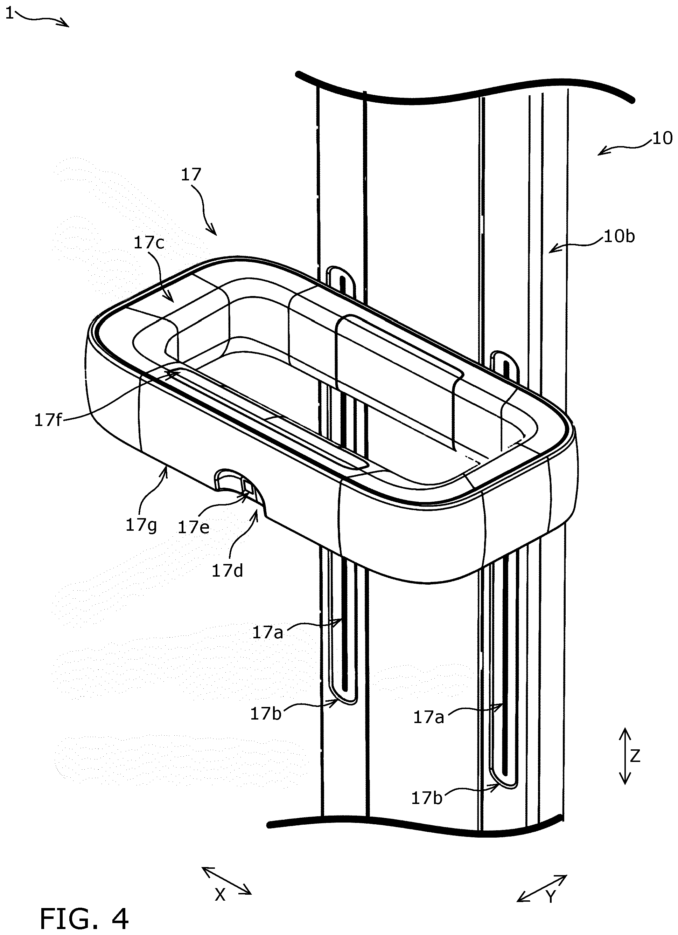

Analog ist am zweiten, rechten Seitenelement 10c des Rahmens 10 ein zweites, rechtes höhenverstellbares Handelement 17 angeordnet, siehe zum Beispiel Figur 4, welches dort wie zuvor beschrieben mittels eines Höhenverstellelements 17a durch Durchgangsöffnungen 17b hindurch in der vertikalen Richtung Z bewegt werden kann. Ein Griffelement 17c des zweiten, rechten Seitenelements 10c des Rahmens 10 weist eine Fingermulde 17d, einen zweiten, rechten optischen Handsensor 17e sowie eine zweite, rechte Handelektrode 17f, 17g auf, welche ein erstes, oberes Handelektrodenelement 17f und ein zweites, unteres Handelektrodenelement 17g aufweist.

Das Dachelement 10d des Rahmens 10 weist in der Querrichtung Y mittig und in der vertikalen Richtung Z an seiner Unterseite der Person 2 zugewandt einen Größenmessungssensor 18 auf, welcher in der vertikalen Richtung Z nach unten zum Bodenelement 10a des Rahmens 10 hin ausgerichtet ist. Steht nun die Person 2 innerhalb des Rahmens 10 auf dem Bodenelement 10a des Rahmens 10, so kann ein Signal vom Größenmessungssensor 18 zur Person 2 hin ausgesendet und eine Reflexion des Signals sensorisch erfasst werden. Aus dem Laufzeitunterschied des ausgesendeten Signals und dessen empfangener Reflexion kann ein Abstand zur Person 2 bestimmt werden. In Kenntnis des Abstands zwischen Größenmessungssensor 18 und der in der vertikalen Richtung Z nach oben zeigenden Oberfläche des Bodenelements 10a des Rahmens 10 kann als Differenz die Größe der Person 2 von der Steuerungseinheit der Vorrichtung 1 berechnet bzw. bestimmt werden. Auch diese Information kann als Gesundheitsparameter der Person 2 mittels des Anzeigeelements 11 angezeigt sowie weiterverarbeitet bzw. in Kombination mit anderen Gesundheitsparametern verwendet werden.

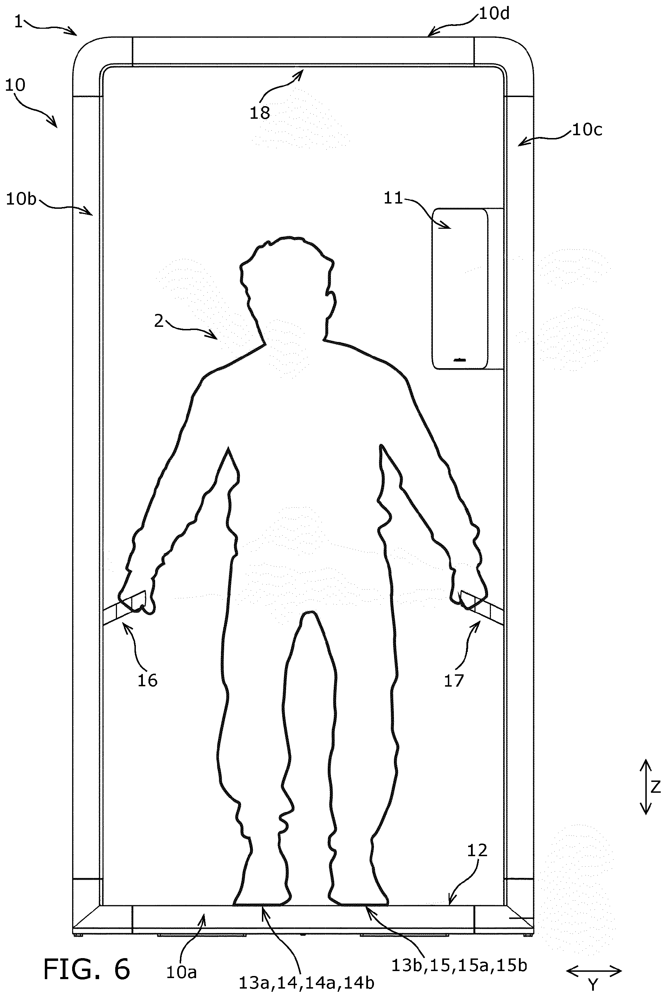

Die bestimmungsgemäße Verwendung der Vorrichtung 1 erfolgt nun derart, dass die beiden höhenverstellbaren Handelemente 16, 17 aufgrund ihres Eigengewichts im ungenutzten Zustand der Vorrichtung 1 in der ersten, unteren Position ruhen. Möchte nun eine Person die Vorrichtung 1 zur Bestimmung von Gesundheitsparametern nutzen und tritt mit den nackten Füßen, d.h. Bafuß, auf die beiden Fußelektroden 14, 15 des Bodenelements 10a des Rahmens 10, so ist die erste, untere Position der beiden höhenverstellbaren Handelemente 16, 17 konstruktiv derart gewählt, dass die Person 2 eine gebückte bzw. eine gebeugte Körperhaltung einnehmen bzw. in die Knie gehen muss, um die Griffelemente 16c, 17c der beiden höhenverstellbaren Handelemente 16, 17 mit den Händen greifen zu können, siehe zum Beispiel Figur 6.

Hat die Person 2 mit den Händen die beiden Griffelemente 16c, 17c der beiden höhenverstellbaren Handelemente 16, 17 wie zuvor beschrieben gegriffen, wird sich die Person 2 intuitiv Aufrichten, d. h. eine aufrechte Körperhaltung in der vertikalen Richtung Z einnehmen, siehe zum Beispiel Figur 7. Diese beiden Schritte können gegebenenfalls durch entsprechende Anweisungen seitens der Vorrichtung 1 unterstützt werden, welche akustisch durch Sprachausgabe und bzw. oder durch angezeigte Anweisungen in Textform und bzw. oder mittels Symbolen auf dem Anzeige-/Bedienelement 11 ausgegeben werden können.

Durch das Aufrichten der Person 2 werden die beiden gegriffenen Griffelemente 16c, 17c der beiden höhenverstellbaren Handelemente 16, 17 jeweils in der vertikalen Richtung Z entlang der Durchgangsöffnungen 16b, 17b der beiden Seitenelemente 10b, 10c des Rahmens 10 nach oben bewegt, wobei die Person 2 lediglich das Eigengewicht der beiden höhenverstellbaren Handelemente 16, 17 bewegen muss. Intuitiv lässt die Person 2 dabei die Arme länglich gestreckt. Die beiden Seitenelemente 10b, 10c des Rahmens 10 sind vorbestimmt derart in der Querrichtung Y angeordnet, dass sich durch das Aufrichten der Person 2 ein vorbestimmter und insbesondere ein reproduzierbarer Winkel zwischen dem Oberkörper und den Armen der Person 2 ergibt. Hierdurch wird ein elektrischer Kontakt zwischen Armen und Oberkörper der Person vermieden und gleichzeitig ein vorbestimmter Winkel erreicht.

In dieser vorbestimmten, definierten und reproduzierbaren Körperhaltung, welche die Person einfach, schnell und insbesondere intuitiv einnimmt, können nun gleichzeitig oder zeitlich unmittelbar aufeinanderfolgend verschiedene Messungen wie zuvor beschrieben von der Vorrichtung 1 durchgeführt werden. Zum einen können die insgesamt acht Elektroden der Fußelektroden 14, 15 und der Handelektroden 16f, 16g, 17f, 17g zur Durchführung einer octopolaren bioelektrischen Impedanzanalyse verwendet werden. Einige der Elektroden der Fußelektroden 14, 15 und der Handelektroden 16f, 16g, 17f, 17g können zuvor oder anschließend zur Durchführung einer Elektrokardiografie verwendet werden. Parallel können die optischen Fußsensoren 13a, 13b sowie die optischen Handsensoren 16e, 17e zur Durchführung einer Multi-Site-Photoplethysmographie (MPPG) verwendet werden. Parallel kann mittels der Waageeinrichtung 12 das Gewicht der Person 2 bestimmt werden. Parallel kann mittels des Größenmessungssensors 18 die Größe der Person 2 bestimmt werden.

Die Verarbeitung der entsprechenden Sensorwerte zur Ermittlung der entsprechenden Gesundheitsparameter sowie von Kombinationen einiger oder aller der bestimmten Gesundheitsparameter kann seitens der Steuerungseinheit der Vorrichtung 1 erfolgen. Die entsprechenden Ergebnisse können auf dem Anzeige-/Bedienelement 11 der Person ausgegeben oder der Person 2 zusätzlich oder alternativ zum Beispiel mittels einer App auf einem Smartphone der Person 2 zur Verfügung gestellt werden. Insbesondere durch die bestimmen Gesundheitsparameter des Pulses und der Sauerstoffsättigung des Blutes der Person 2 mittels der Multi-Site-Photoplethysmographie in Verbindung mit einem Elektrokardiogramm können auch Aussagen zu Blutdruck, Arteriosklerose und Gefäßalterung der Person 2 durchgeführt werden.

Das Anzeige-/Bedienelement 11 kann insbesondere als Touchdisplay 11 realisiert sein, sodass die Bedienung über eine graphische Oberfläche erfolgen kann. Über das Touchdisplay 11 kann der Person 2 als Nutzer die Handhabung der Vorrichtung 1 erklärt werden. Ebenso kann die Person 2 als Nutzer über das Touchdisplay 11 personenbezogene Daten eingeben und den Messvorgang starten. Die Person 2 kann durch Angaben auf dem Touchdisplay 11 auch vor Kontraindikatoren gewarnt werden. Somit ist keine Betreuung durch Fachpersonal bzw. durch geschultes Personal erforderlich, um die Vorrichtung 1 bestimmungsgemäß nutzen zu können.

Die Ergebnisse der bestimmten Gesundheitsparameter können digital so verarbeitet werden, dass leicht verständliche Grafiken auf dem Touchdisplay 11 nach Beendigung der Messung dargestellt werden können. Dies kann zusätzlich oder alternativ auch zum Beispiels seitens einer App auf dem Smartphone der Person 2 erfolgen. Beispielsweise können durch die Auswertung der Herzratenvariabilität, des Pulses, des Blutdrucks und der Körperzusammensetzung Aussage über den Stresslevel getroffen werden. Parameter der Körperzusammensetzung können beispielsweise das Körperwasser, der segmental ausgewertete Muskelanteil, das Körperfett und die fettfreie Masse sein. Der Wasseranteil im Körper kann in einen intrazellulären und einen extrazellulären Anteil aufgeteilt werden. Weiter kann aus den gewonnenen Daten ein individueller Trainings- und Ernährungsplan sowie Entspannungsübungen der Person 2 in einer dazugehörigen Software erstellt werden.

Die Vorrichtung 1 kann dahingehend verwendet werden, eine ganzheitliche Betrachtung des Körpers der Person 2 vorzunehmen, was für eine zielgerichtete Trainingssteuerung wichtig sein kann. So können Krankheiten, die zum Tode führen können wie zum Beispiel der plötzliche Herztod oder Arteriosklerose, präventiv vermieden werden. Insbesondere bei Hochleistungssportlern, die einem strengen Trainings- und Ernährungsplan folgen, können gesundheitliche Beeinträchtigungen des Herzens auftreten.

Eine regelmäßige Wiederholung der Selbstvermessung der Person 2 mittels der Vorrichtung 1 dient der Kontrolle der Trainings- und Erholungsphasen, bietet die Stressüberwachung und -minderung, sowie Wissen über Leistungsgrenzen im Zusammenhang mit der Körperzusammensetzung. Daher ist es von Vorteil, dass mittels der Vorrichtung 1 ein Gerät zur schnellen Selbstvermessung der Person 2 ohne Personalaufwand mit benutzerfreundlicher Bedienung geschaffen werden kann.

Um möglichst aussagekräftige Messergebnisse zu erreichen, welche möglichst viele Rückschlüsse auf die Gesundheit des Körpers der zu vermessenden Person 2 ermöglichen, kann es besonders vorteilhaft sein, mehrere Messverfahren zu kombinieren und die einzelnen Sensoren so anzuordnen, dass möglichst eine einfache Bedienung ermöglicht wird und dass die Körperhaltung der Person 2 während der Messung möglichst reproduzierbar ist. Dies kann durch die erfindungsgemäße Vorrichtung 1 erreicht werden.

### BEZUGSZEICHENLISTE (Teil der Beschreibung)

- X: Längsrichtung; Tiefe; Länge
- Y: Querrichtung; Breite
- Z: vertikale Richtung; Höhe
- X, Y: Horizontale; horizontale Ebene

- 1: Vorrichtung zur Bestimmung wenigstens eines Gesundheitsparameters einer Person 2; multifunktionale Messvorrichtung
- 10: Rahmen; Rahmenelement
- 10a: Bodenelement des Rahmens 10
- 10b: erstes, linkes Seitenelement des Rahmens 10
- 10c: zweites, rechtes Seitenelement des Rahmens 10
- 10d: Dachelement des Rahmens 10
- 11: Anzeigeelement; Bedienelement; Anzeige-/Bedienelement; Touchdisplay
- 12: Waageeinrichtung; Vorrichtung zum Wiegen
- 13a: erster, linker optischer Fußsensor; optischer Sensor für Fuß
- 13b: zweiter, rechter optischer Fußsensor; optischer Sensor für Fuß
- 14: erste, linke Fußelektrode
- 14a: erstes, vorderes Fußelektrodenelement der ersten, linken Fußelektrode 14 für Ballen
- 14b: zweites, hinteres Fußelektrodenelement der ersten, linken Fußelektrode 14 für Fersen
- 15: zweite, rechte Fußelektrode
- 15a: erstes, vorderes Fußelektrodenelement der zweiten, rechten Fußelektrode 15 für Ballen
- 15b: zweites, hinteres Fußelektrodenelement der zweiten, rechten Fußelektrode 15 für Fersen
- 16: erstes, linkes höhenverstellbares Handelement
- 16a: Höhenverstellelement bzw. Höhenverstellung des ersten, linken höhenverstellbaren Handelements 16
- 16b: Durchgangsöffnungen des ersten, linken Seitenelements 10b des Rahmens 10 für das Höhenverstellelement 16a des ersten, linken höhenverstellbaren Handelements 16
- 16c: Griffelement, Griffstück bzw. Handgriff des ersten, linken höhenverstellbaren Handelements 16
- 16d: Fingermulde des ersten, linken höhenverstellbaren Handelements 16
- 16e: erster, linker optischer Handsensor bzw. optischer Sensor für Hand des ersten, linken höhenverstellbaren Handelements 16
- 16f: erstes, oberes Handelektrodenelement der ersten, linken Handelektrode 16f, 16g des ersten, linken höhenverstellbaren Handelements 16
- 16g: zweites, unteres Handelektrodenelement der ersten, linken Handelektrode 16f, 16g des ersten, linken höhenverstellbaren Handelements 16
- 17: zweites, rechtes höhenverstellbares Handelement
- 17a: Höhenverstellelement bzw. Höhenverstellung des zweiten, rechten höhenverstellbaren Handelements 17
- 17b: Durchgangsöffnungen des zweiten, rechten Seitenelements 10c des Rahmens 10 für das Höhenverstellelement 17a des zweiten, rechten höhenverstellbaren Handelements 17
- 17c: Griffelement, Griffstück bzw. Handgriff des zweiten, rechten höhenverstellbaren Handelements 17
- 17d: Fingermulde des zweiten, rechten höhenverstellbaren Handelements 17
- 17e: zweiter, rechter optischer Handsensor bzw. optischer Sensor für Hand des zweiten, rechten höhenverstellbaren Handelements 17
- 17f: erstes, oberes Handelektrodenelement der zweiten, rechten Handelektrode 17f, 17g des zweiten, rechten höhenverstellbaren Handelements 17
- 17g: zweites, unteres Handelektrodenelement der zweiten, rechten Handelektrode 17f, 17g des zweiten, rechten höhenverstellbaren Handelements 17
- 18: Größenmessungssensor; Sensoren Höhenmessung

- 2: (zu vermessende) Person; biologisches Subjekt

## Patentansprüche

1. Vorrichtung (1) zur Bestimmung wenigstens eines Gesundheitsparameters einer Person (2) mit einer ersten Fußelektrode (14) und einer zweiten Fußelektrode (15), welche ausgebildet sind, jeweils von einem Fuß der Person (2) elektrisch kontaktiert zu werden, und
mit einer ersten Handelektrode (16f, 16g) und einer zweiten Handelektrode (17f, 17g), welche ausgebildet sind, jeweils von einer Hand der Person (2) elektrisch kontaktiert zu werden, wobei die Handelektroden (16f, 16g; 17f, 17g) in der vertikalen Richtung (Z) höhenverstellbar ausgebildet sind,
wobei eine Steuerungseinheit der Vorrichtung (1) ausgebildet ist, mittels der Fußelektroden (14, 15) und mittels der Handelektroden (16f, 16g; 17f, 17g) Sensorwerte zur Durchführung einer bioelektrischen Impedanzanalyse zu erfassen,
wobei
die Handelektroden (16f, 16g; 17f, 17g) ferner ausgebildet sind, jeweils von einer Hand der Person (2) gegriffen zu werden,
wobei die Handelektroden (16f, 16g; 17f, 17g) ferner ausgebildet sind,
in der vertikalen Richtung (Z) eine erste untere Position, die einen unteren Endanschlag der Handelektrode (16f, 16g; 17f, 17g) darstellt, aufgrund ihres Eigengewichts und der Schwerkraft von alleine einzunehmen und dort zu ruhen, so dass die Handelektroden (16f, 16g; 17f, 17g) von der Person in einer gebeugten Körperhaltung zu greifen sind, und
von der Person in der vertikalen Richtung (Z) in eine zweite, obere Position bewegt zu werden, in welcher die Person (2) in einer aufrechten Körperhaltung steht, und wobei vorzugsweise die Steuerungseinheit der Vorrichtung (1) ausgebildet ist, die bioelektrische Impedanzanalyse durchzuführen.

2. Vorrichtung (1) nach Anspruch 1, **dadurch gekennzeichnet, dass**
die Handelektroden (16f, 16g; 17f, 17g) ausgebildet sind, in der vertikalen Richtung (Z) jeweils oder gemeinsam mit einem Höhenverstellelement (16a; 17a) mit bewegt zu werden,
wobei das Höhenverstellelement (16a; 17a) ausgebildet ist, eine Bewegung der Handelektroden (16f, 16g; 17f, 17g) in der vertikalen Richtung (Z) nach unten, vorzugsweise mittels wenigstens eines in der vertikalen Richtung (Z) ausgerichteten Bewegungsdämpfungselements, zu dämpfen.

3. Vorrichtung (1) nach Anspruch 1 oder 2, **dadurch gekennzeichnet, dass**
die Handelektroden (16f, 16g; 17f, 17g) einander in der Querrichtung (Y), vorzugsweise diametral zur Person (2), gegenüberliegend seitlich des Paares von Fußelektroden (14; 15) angeordnet sind.

4. Vorrichtung (1) nach einem der vorangehenden Ansprüche, **dadurch gekennzeichnet, dass**
die Handelektroden (16f, 16g; 17f, 17g) in der Querrichtung (Y) derart beabstandet sind, so dass die Person (2) in der zweiten, oberen Position eine seitlich abgespreizte Armhaltung einnimmt.

5. Vorrichtung (1) nach einem der vorangehenden Ansprüche, **dadurch gekennzeichnet, dass**
die erste Handelektrode (16f, 16g) ein erstes Handelektrodenelement (16f) und ein zweites Handelektrodenelement (16g) aufweist, und/oder
die zweite Handelektrode (17f, 17g) ein erstes Handelektrodenelement (17f) und ein zweites Handelektrodenelement (17g) aufweist, und/oder
die erste Fußelektrode (14) ein erstes Fußelektrodenelement (14a) und ein zweites Fußelektrodenelement (14b) aufweist und/oder
die zweite Fußelektrode (15) ein erstes Fußelektrodenelement (15a) und ein zweites Fußelektrodenelement (15b) aufweist.

6. Vorrichtung (1) nach Anspruch 5, **dadurch gekennzeichnet, dass**
das erste Handelektrodenelement (16f) und das zweite Handelektrodenelement (16g) der ersten Handelektrode (16f, 16g) einander in der vertikalen Richtung (Z), vorzugsweise diametral, gegenüberliegend, vorzugsweise an einem ersten höhenverstellbaren Handelement (16), angeordnet sind, und/oder
das erste Handelektrodenelement (17f) und das zweite Handelektrodenelement (17g) der zweiten Handelektrode (17f, 17g) einander in der vertikalen Richtung (Z), vorzugsweise diametral, gegenüberliegend, vorzugsweise an einem zweiten höhenverstellbaren Handelement (17), angeordnet sind.

7. Vorrichtung (1) nach einem der vorangehenden Ansprüche, **dadurch gekennzeichnet, dass**
die Steuerungseinheit der Vorrichtung (1) ferner ausgebildet ist, ein Elektrokardiogramm zu erstellen.

8. Vorrichtung (1) nach einem der vorangehenden Ansprüche, **gekennzeichnet durch**
wenigstens einen optischen Sensor (13a, 13b, 16e, 17e), welcher ausgebildet ist, Sensorwerte zur Durchführung einer Photoplethysmographie und/oder einer Pulsoxymetrie zu erfassen,
wobei vorzugsweise die Steuerungseinheit der Vorrichtung (1) ferner ausgebildet ist, die Photoplethysmographie und/oder die Pulsoxymetrie durchzuführen.

9. Vorrichtung (1) nach Anspruch 8, **gekennzeichnet durch**
einen ersten optischen Handsensor (16e), welcher an einem ersten höhenverstellbaren Handelements (16), vorzugsweise in der vertikalen Richtung (Z) zwischen einem ersten Handelektrodenelement (16f) und einem zweiten Handelektrodenelement (16g) der ersten Handelektrode (16f, 16g) und/oder in der Querrichtung (Y) zur Person (2) hinzeigend, angeordnet und ausgebildet ist, zur Durchführung einer Photoplethysmographie und/oder einer Pulsoxymetrie verwendet zu werden, und/oder
einen zweiten optischen Handsensor (17e), welcher an einem zweiten höhenverstellbaren Handelements (17), vorzugsweise in der vertikalen Richtung (Z) zwischen einem ersten Handelektrodenelement (17f) und einem zweiten Handelektrodenelement (17g) der zweiten Handelektrode (17f, 17g) und/oder in der Querrichtung (Y) zur Person (2) hinzeigend, angeordnet und ausgebildet ist, zur Durchführung einer Photoplethysmographie und/oder einer Pulsoxymetrie verwendet zu werden, und/oder
einen ersten optischen Fußsensor (13a), welcher vorzugsweise innerhalb der ersten Fußelektrode (14), besonders vorzugsweise innerhalb des ersten Fußelektrodenelements (14a) oder innerhalb des zweiten Fußelektrodenelements (14b) der ersten Fußelektrode (14), angeordnet und ausgebildet ist, zur Durchführung einer Photoplethysmographie und/oder einer Pulsoxymetrie verwendet zu werden, und/oder
einen zweiten optischen Fußsensor (13b), welcher vorzugsweise innerhalb der zweiten Fußelektrode (15), besonders vorzugsweise innerhalb des ersten Fußelektrodenelements (15a) oder innerhalb des zweiten Fußelektrodenelements (15b) der zweiten Fußelektrode (15), angeordnet und ausgebildet ist, zur Durchführung einer Photoplethysmographie und/oder einer Pulsoxymetrie verwendet zu werden,
wobei vorzugsweise der erste optische Handsensor (16e) innerhalb einer Fingermulde (16d) des ersten höhenverstellbaren Handelements (16) angeordnet ist und/oder
vorzugsweise der zweite optische Handsensor (17e) innerhalb einer Fingermulde (17d) des zweiten höhenverstellbaren Handelements (17) angeordnet ist.

10. Vorrichtung (1) nach einem der vorangehenden Ansprüche, **gekennzeichnet durch** einen Rahmen (10)
mit einem Bodenelement (10a), welches die Fußelektroden (14; 15) aufweist,
mit einem ersten Seitenelement (10b), welches die erste Handelektrode (16f, 16g), vorzugsweise ein erstes höhenverstellbares Handelement (16) mit der erste Handelektrode (16f, 16g), in der vertikalen Richtung (Z) zwischen der ersten, unteren Position und der zweiten, oberen Position höhenverstellbar aufweist, und
mit einem zweiten Seitenelement (10c), welches die zweite Handelektrode (17f, 17g), vorzugsweise ein zweites höhenverstellbares Handelement (17) mit der zweiten Handelektrode (17f, 17g), in der vertikalen Richtung (Z) zwischen der ersten, unteren Position und der zweiten, oberen Position höhenverstellbar aufweist.

11. Vorrichtung (1) nach Anspruch 10, **dadurch gekennzeichnet, dass**
der Rahmen (10) ein Dachelement (10d) aufweist,
wobei das Bodenelement (10a), das erste Seitenelement (10b), das zweite Seitenelement (10c) und das Dachelement (10d) eine zumindest im Wesentlichen rechteckige Form bilden, welche ausgebildet ist, die Person in der aufrechten Körperhaltung in sich aufzunehmen.

12. Vorrichtung (1) nach einem der vorangehenden Ansprüche, **gekennzeichnet durch**
einen Größenmessungssensor (18), welcher in der vertikalen Richtung (Z) derart oberhalb der Fußelektroden (14, 15) angeordnet und zu den Fußelektroden (14, 15) hin ausgerichtet ist, so dass ein Sensorwert, welcher einen Abstand zur Person (2) repräsentiert, erfasst werden kann,
wobei vorzugsweise die Steuerungseinheit der Vorrichtung (1) ausgebildet ist, aus dem Sensorwert des Größenmessungssensors (18) die Größe der Person (2) zu bestimmen, und/oder
wobei vorzugsweise der Größenmessungssensor (18) an dem Dachelement (10d) des Rahmens (10) angeordnet ist.

13. Vorrichtung (1) nach einem der vorangehenden Ansprüche, **gekennzeichnet durch**
eine Waageeinrichtung (12), welche ausgebildet ist, die Person (2) auf sich aufzunehmen und einen das Gewicht der Person (2) repräsentierenden Sensorwert zu erfassen,
wobei vorzugsweise die Steuerungseinheit der Vorrichtung (1) ferner ausgebildet ist, aus dem Sensorwert der Waageeinrichtung (12) das Gewicht der Person (2) zu bestimmen.

14. Vorrichtung (1) nach Anspruch 13, **dadurch gekennzeichnet, dass**
die Waageeinrichtung (12) in der vertikalen Richtung (Z) unterhalb des Paares von Fußelektroden (14; 15) angeordnet ist.

15. Vorrichtung (1) nach einem der vorangehenden Ansprüche, **gekennzeichnet durch**
ein Anzeigeelement (11) oder ein Anzeige-/Bedienelement (11), welches ausgebildet ist, der Person (2) wenigstens einen mittels der bioelektrischen Impedanzanalyse bestimmten Gesundheitsparameters der Person (2) anzuzeigen, und/oder
ein Bedienelement (11) oder ein Anzeige-/Bedienelement (11), welches ausgebildet ist, wenigstens eine Eingabe der Person (2) zu erfassen.

## Claims

1. Device (1) for determining at least one health parameter of a person (2)
comprising a first foot electrode (14) and a second foot electrode (15), which are configured to be electrically contacted by a respective foot of the person (2), and
comprising a first hand electrode (16f, 16g) and a second hand electrode (17f, 17g), which are configured to be electrically contacted by a respective hand of the person (2),
wherein the hand electrodes (16f, 16g; 17f, 17g) are designed to be height-adjustable in the vertical direction (Z),
wherein a control unit of the device (1) is configured to use the foot electrodes (14, 15) and the hand electrodes (16f, 16g; 17f, 17g) to acquire sensor values for performing a bioelectrical impedance analysis,
wherein the hand electrodes (16f, 16g; 17f, 17g) are further configured to be grasped by one hand of the person (2) each,
wherein the hand electrodes (16f, 16g; 17f, 17g) are further configured to
assume, in the vertical direction (Z), a first lower position-which represents a lower end stop of the hand electrode (16f, 16g; 17f, 17g), due to their own weight and the force of gravity, and to remain there, so that the hand electrodes (16f, 16g; 17f, 17g) can be grasped by the person in a bent posture, and
to be moved by the person in the vertical direction (Z) into a second, upper position, in which the person (2) stands in an upright posture, and
wherein the control unit of the device (1) is preferably configured to perform bioelectrical impedance analysis.

2. Device (1) according to claim 1, **characterized in that**
the hand electrodes (16f, 16g; 17f, 17g) are configured to be moved in the vertical direction (Z) individually or jointly with a height-adjustment element (16a; 17a),
wherein the height-adjustment element (16a; 17a) is configured to dampen downward movement of the hand electrodes (16f, 16g; 17f, 17g) in the vertical direction (Z), preferably by means of at least one motion-damping element aligned in the vertical direction (Z).

3. Device (1) according to claim 1 or 2, **characterized in that**
the hand electrodes (16f, 16g; 17f, 17g) are arranged opposite one another in the transverse direction (Y), preferably diametrically opposite the person (2), on either side of the pair of foot electrodes (14; 15).

4. Device (1) according to any one of the preceding claims, **characterized in that**
the hand electrodes (16f, 16g; 17f, 17g) are spaced apart in the transverse direction (Y) such that the person (2) assumes a laterally spread arm position in the second, upper position.

5. A device (1) according to any one of the preceding claims, **characterized in that**
the first hand electrode (16f, 16g) comprises a first hand electrode element (16f) and a second hand electrode element (16g), and/or
the second hand electrode (17f, 17g) comprises a first hand electrode element (17f) and a second hand electrode element (17g), and/or
the first foot electrode (14) comprises a first foot electrode element (14a) and a second foot electrode element (14b), and/or
the second foot electrode (15) comprises a first foot electrode element (15a) and a second foot electrode element (15b).

6. Apparatus (1) according to claim 5**, characterized in that**
the first hand electrode element (16f) and the second hand electrode element (16g) of the first hand electrode (16f, 16g) are arranged opposite one another in the vertical direction (Z), preferably diametrically opposite, preferably on a first height-adjustable hand element (16), and/or
the first hand electrode element (17f) and the second hand electrode element (17g) of the second hand electrode (17f, 17g) are arranged opposite one another in the vertical direction (Z), preferably diametrically, preferably on a second height-adjustable hand element (17).

7. A device (1) according to any one of the preceding claims, **characterized in that**
the control unit of the device (1) is further configured to generate an electrocardiogram.

8. A device (1) according to any one of the preceding claims**, characterized by**
at least one optical sensor (13a, 13b, 16e, 17e), which is configured to detect sensor values for performing photoplethysmography and/or pulse oximetry,
wherein, preferably, the control unit of the device (1) is further configured to perform the photoplethysmography and/or the pulse oximetry.

9. Device (1) according to claim 8, **characterized by**
a first optical hand sensor (16e), which is arranged on a first height-adjustable hand element (16), preferably in the vertical direction (Z) between a first hand electrode element (16f) and a second hand electrode element (16g) of the first hand electrode (16f, 16g) and/or pointing toward the person (2) in the transverse direction (Y), and is configured to be used for performing photoplethysmography and/or pulse oximetry, and/or
a second optical hand sensor (17e), which is arranged on a second height-adjustable hand element (17), preferably in the vertical direction (Z) between a first hand electrode element (17f) and a second hand electrode element (17g) of the second hand electrode (17f, 17g) and/or pointing toward the person (2) in the transverse direction (Y), and is configured to be used for performing photoplethysmography and/or pulse oximetry, and/or
a first optical foot sensor (13a), which is preferably arranged within the first foot electrode (14), more particularly preferably within the first foot electrode element (14a) or within the second foot electrode element (14b) of the first foot electrode (14), arranged and configured to be used for performing photoplethysmography and/or pulse oximetry, and/or
a second optical foot sensor (13b), which is preferably arranged and configured within the second foot electrode (15), most preferably within the first foot electrode element (15a) or within the second foot electrode element (15b) of the second foot electrode (15), and is configured to be used for performing photoplethysmography and/or pulse oximetry,
wherein preferably the first optical hand sensor (16e) is arranged within a finger recess (16d) of the first height-adjustable hand element (16) and/or
preferably the second optical hand sensor (17e) is arranged within a finger recess (17d) of the second height-adjustable hand element (17).

10. A device (1) according to any one of the preceding claims, **characterized by**
a frame (10)
with a base element (10a) comprising the foot electrodes (14; 15),
with a first side element (10b) comprising the first hand electrode (16f, 16g), preferably a first height-adjustable hand element (16) with the first hand electrode (16f, 16g), which is height-adjustable in the vertical direction (Z) between the first, lower position and the second, upper position, and
a second side element (10c) comprising the second hand electrode (17f, 17g), preferably a second height-adjustable hand element (17) with the second hand electrode (17f, 17g), which is height-adjustable in the vertical direction (Z) between the first, lower position and the second, upper position.

11. Apparatus (1) according to claim 10, **characterized in that**
the frame (10) includes a roof element (10d),
wherein the base element (10a), the first side element (10b), the second side element (10c), and the roof element (10d) form an at least substantially rectangular shape configured to accommodate the person in an upright posture.

12. A device (1) according to any one of the preceding claims, **characterized by**
a height measurement sensor (18) which is arranged in the vertical direction (Z) above the foot electrodes (14, 15) and is aligned with the foot electrodes (14, 15) so that a sensor value representing a distance to the person (2) can be detected,
wherein, preferably, the control unit of the device (1) is configured to determine the height of the person (2) from the sensor value of the height-measuring sensor (18), and/or
wherein, preferably, the height measurement sensor (18) is arranged on the roof element (10d) of the frame (10).

13. Device (1) according to one of the preceding claims, **characterized by**
a weighing device (12) configured to support the person (2) and to detect a sensor value representing the weight of the person (2),
wherein, preferably, the control unit of the device (1) is further configured to determine the weight of the person (2) based on the sensor value from the weighing device (12).

14. A device (1) according to claim 13, **characterized in that**
the weighing device (12) is arranged in the vertical direction (Z) below the pair of foot electrodes (14; 15).

15. Apparatus (1) according to any one of the preceding claims, **characterized by**
a display element (11) or a display/control element (11) configured to display to the person (2) at least one health parameter of the person (2) determined by means of bioelectrical impedance analysis, and/or
a control element (11) or a display/control element (11) configured to detect at least one input from the person (2).

## Revendications

1. Dispositif (1) destiné à déterminer au moins un paramètre de santé d'une personne (2)
comprenant une première électrode de pied (14) et une deuxième électrode de pied (15), qui sont conçues pour être mises en contact électrique respectivement avec un pied de la personne (2), et
comprenant une première électrode de main (16f, 16g) et d'une deuxième électrode de main (17f, 17g), qui sont conçues pour être mises en contact électrique respectivement avec une main de la personne (2),
les électrodes de main (16f, 16g ; 17f, 17g) étant réglables en hauteur dans la direction verticale (Z),
une unité de commande du dispositif (1) étant conçue pour enregistrer, au moyen des électrodes de pied (14, 15) et au moyen des électrodes de main (16f, 16g ; 17f, 17g) des valeurs de capteur pour effectuer une analyse d'impédance bioélectrique,
les électrodes de main (16f, 16g ; 17f, 17g) étant en outre conçues pour être saisies chacune par une main de la personne (2),
les électrodes de main (16f, 16g ; 17f, 17g) sont en outre conçues pour occuper de manière autonome,
dans la direction verticale (Z), une première position inférieure qui correspond à une butée d'extrémité inférieure de l'électrode de main (16f, 16g ; 17f, 17g), sous l'effet de leur propre poids et de la gravité, et d'y rester, de sorte que les électrodes de main (16f, 16g ; 17f, 17g) puissent être saisies par la personne dans une posture courbée, et
soient déplacées par la personne dans la direction verticale (Z) vers une deuxième position supérieure, dans laquelle la personne (2) se tient dans une posture droite, et
l'unité de commande du dispositif (1) étant de préférence conçue pour effectuer l'analyse d'impédance bioélectrique.

2. Dispositif (1) selon la revendication 1, **caractérisé en ce que**
les électrodes portatives (16f, 16g ; 17f, 17g) sont conçues pour être déplacées dans la direction verticale (Z) respectivement ou conjointement avec un élément de réglage en hauteur (16a ; 17a),
l'élément de réglage en hauteur (16a ; 17a) est conçu pour amortir un mouvement des électrodes de main (16f, 16g ; 17f, 17g) dans la direction verticale (Z) vers le bas, de préférence au moyen d'au moins un élément d'amortissement de mouvement orienté dans la direction verticale (Z).

3. Dispositif (1) selon la revendication 1 ou 2, **caractérisé en ce que**
les électrodes de main (16f, 16g ; 17f, 17g) sont disposées face à face dans la direction transversale (Y), de préférence diamétralement opposées à la personne (2), sur les côtés de la paire d'électrodes de pied (14 ; 15).

4. Dispositif (1) selon l'une des revendications précédentes, **caractérisé en ce que**
les électrodes de main (16f, 16g ; 17f, 17g) sont espacées dans la direction transversale (Y) de telle sorte que la personne (2) adopte, dans la deuxième position supérieure, une posture des bras écartés latéralement.

5. Dispositif (1) selon l'une des revendications précédentes, **caractérisé en ce que**
la première électrode de main (16f, 16g) comporte un premier élément d'électrode de main (16f) et un deuxième élément d'électrode de main (16g), et/ou
la deuxième électrode de main (17f, 17g) comporte un premier élément d'électrode de main (17f) et un deuxième élément d'électrode de main (17g), et/ou
la première électrode de pied (14) comporte un premier élément d'électrode de pied (14a) et un deuxième élément d'électrode de pied (14b), et/ou
la deuxième électrode de pied (15) comporte un premier élément d'électrode de pied (15a) et un deuxième élément d'électrode de pied (15b).

6. Dispositif (1) selon la revendication 5, **caractérisé en ce que**
le premier élément d'électrode de main (16f) et le deuxième élément d'électrode de main (16g) de la première électrode de main (16f, 16g) sont disposés l'un en face de l'autre dans la direction verticale (Z), de préférence de manière diamétralement opposée, de préférence sur un premier élément de poignée réglable en hauteur (16), et/ou
le premier élément d'électrode manuelle (17f) et le deuxième élément d'électrode manuelle (17g) de la deuxième électrode manuelle (17f, 17g) sont disposés en vis-à-vis dans la direction verticale (Z), de préférence de manière diamétralement opposée, de préférence sur un deuxième élément manuel réglable en hauteur (17).

7. Dispositif (1) selon l'une des revendications précédentes, **caractérisé en ce que**
l'unité de commande du dispositif (1) est en outre conçue pour générer un électrocardiogramme.

8. Dispositif (1) selon l'une des revendications précédentes, **caractérisé par**
au moins un capteur optique (13a, 13b, 16e, 17e), qui est conçu pour enregistrer des valeurs de capteur en vue de la réalisation d'une photopléthysmographie et/ou d'une oxymétrie de pouls,
l'unité de commande du dispositif (1) étant de préférence en outre conçue pour réaliser la photopléthysmographie et/ou l'oxymétrie de pouls.

9. Dispositif (1) selon la revendication 8, **caractérisé par**
un premier capteur optique de main (16e), qui est disposé sur un premier élément de main (16) réglable en hauteur, de préférence dans la direction verticale (Z) entre un premier élément d'électrode de main (16f) et un deuxième élément d'électrode de main (16g) de la première électrode de main (16f, 16g) et/ou dans la direction transversale (Y) en direction de la personne (2), et est conçu pour être utilisé pour réaliser une photopléthysmographie et/ou une oxymétrie de pouls, et/ou
un deuxième capteur optique de main (17e), qui est disposé sur un deuxième élément de main réglable en hauteur (17), de préférence dans la direction verticale (Z) entre un premier élément d'électrode de main (17f) et un deuxième élément d'électrode de main (17g) de la deuxième électrode de main (17f, 17g) et/ou orienté dans la direction transversale (Y) vers la personne (2), est agencé et conçu pour être utilisé pour réaliser une photopléthysmographie et/ou une oxymétrie de pouls, et/ou
un premier capteur optique de pied (13a), qui est de préférence disposé à l'intérieur de la première électrode de pied (14), de préférence à l'intérieur du premier élément d'électrode de pied (14a) ou à l'intérieur du deuxième élément d'électrode de pied (14b) de la première électrode de pied (14), est agencé et conçu pour être utilisé pour la réalisation d'une photopléthysmographie et/ou d'une oxymétrie de pouls, et/ou
un deuxième capteur optique de pied (13b), qui est de préférence disposé et conçu à l'intérieur de la deuxième électrode de pied (15), de manière particulièrement préférée à l'intérieur du premier élément d'électrode de pied (15a) ou à l'intérieur du deuxième élément d'électrode de pied (15b) de la deuxième électrode de pied (15), et est conçu pour être utilisé pour réaliser une photopléthysmographie et/ou une oxymétrie de pouls,
le premier capteur optique de main (16e) étant de préférence disposé à l'intérieur d'un creux pour doigt (16d) du premier élément de main réglable en hauteur (16) et/ou
de préférence, le deuxième capteur optique de main (17e) est disposé à l'intérieur d'un renfoncement pour doigt (17d) du deuxième élément de main réglable en hauteur (17).

10. Dispositif (1) selon l'une des revendications précédentes, **caractérisé par**
un châssis (10)
comportant un élément de base (10a) qui comporte les électrodes de pied (14 ; 15),
un premier élément latéral (10b) qui comporte la première électrode de main (16f, 16g), de préférence un premier élément de poignée réglable en hauteur (16) comportant la première électrode de poignée (16f, 16g), réglable en hauteur dans la direction verticale (Z) entre une première position inférieure et une deuxième position supérieure, et
comportant un deuxième élément latéral (10c) qui comporte la deuxième électrode de poignée (17f, 17g), de préférence un deuxième élément de poignée réglable en hauteur (17) comportant la deuxième électrode de poignée (17f, 17g), est réglable en hauteur dans la direction verticale (Z) entre la première position inférieure et la deuxième position supérieure.

11. Dispositif (1) selon la revendication 10, **caractérisé en ce que**
le châssis (10) comporte un élément de toit (10d),
l'élément de base (10a), le premier élément latéral (10b), le deuxième élément latéral (10c) et l'élément de toit (10d) formant une forme au moins sensiblement rectangulaire, qui est conçue pour accueillir la personne en position debout.

12. Dispositif (1) selon l'une des revendications précédentes, **caractérisé par**
un capteur de mesure de taille (18), qui est disposé dans la direction verticale (Z) au-dessus des électrodes de pied (14, 15) et orienté vers les électrodes de pied (14, 15) de telle sorte qu'une valeur de capteur, représentant une distance par rapport à la personne (2), puisse être enregistrée,
l'unité de commande du dispositif (1) étant de préférence conçue pour déterminer la taille de la personne (2) à partir de la valeur de capteur du capteur de mesure de taille (18), et/ou
le capteur de taille (18) étant de préférence disposé sur l'élément de toit (10d) du châssis (10).

13. Dispositif (1) selon l'une des revendications précédentes, **caractérisé par**
un dispositif de pesée (12) conçu pour accueillir la personne (2) et pour détecter une valeur de capteur représentant le poids de la personne (2),
l'unité de commande du dispositif (1) étant de préférence également conçue pour déterminer le poids de la personne (2) à partir de la valeur de capteur du dispositif de pesée (12).

14. Dispositif (1) selon la revendication 13, **caractérisé en ce que**
le dispositif de pesée (12) est disposé dans la direction verticale (Z) en dessous de la paire d'électrodes de pied (14 ; 15).

15. Dispositif (1) selon l'une des revendications précédentes, **caractérisé par**
un élément d'affichage (11) ou un élément d'affichage/de commande (11), qui est conçu pour afficher à la personne (2) au moins un paramètre de santé de la personne (2) déterminé au moyen de l'analyse d'impédance bioélectrique, et/ou
un élément de commande (11) ou un élément d'affichage/de commande (11), qui est conçu pour enregistrer au moins une entrée de la personne (2).
